# EUROPEAN PATENT SPECIFICATION

(11) **EP 3 911 354 B1**
(45) Date of publication and mention of the grant of the patent: **19.07.2023**
(21) Application number: 20701951.4
(22) Date of filing: 20.01.2020
(51) Int. Cl.: A61K 38/16, A61K 48/00, C07K 14/00, C12N 15/86, A61P 27/16

(54) **AAV-MEDIATED GENE THERAPY RESTORING THE OTOFERLIN GENE**
AAV-VERMITTELTE GENTHERAPIE ZUR WIEDERHERSTELLUNG DES OTOFERLIN-GENS
THÉRAPIE GÉNIQUE MÉDIÉE PAR AAV RESTAURANT LE GÈNE OTOFERLINE

(30) Priority: 18.01.2019 EP 19305071
(43) Date of publication of application: 24.11.2021
(73) Proprietor: Institut Pasteur, 75015 Paris (FR); CENTRE NATIONAL DE LA RECHERCHE SCIENTIFIQUE (CNRS), 75016 Paris (FR); Institut National de la Santé et de la Recherche Médicale (INSERM), 75013 Paris (FR)
(72) Inventor: SAFIEDDINE, Saaid, 75012 Paris (FR); PETIT, Christine, 75012 Paris (FR)
(74) Representative: Regimbeau
(86) International application number: PCT/EP2020/051283
(87) International publication number: WO 2020/148458

(56) References cited:
- HANAN AL-MOYED ET AL: "A dual-AAV approach restores fast exocytosis and partially rescues auditory function in deaf otoferlin knock-out mice", EMBO MOLECULAR MEDICINE (ONLINE), vol. 11, no. 1, 1 January 2019 (2019-01-01), page e9396, XP055574125, DE ISSN: 1757-4684, DOI: 10.15252/emmm.201809396
- AHMED HENA ET AL: "Emerging Gene Therapies for Genetic Hearing Loss", JOURNAL OF THE ASSOCIATION FOR RESEARCH IN OTOLARYNGOLOGY, SPRINGER, NEW YORK, NY, US, vol. 18, no. 5, 16 August 2017 (2017-08-16), pages 649-670, XP036327909, ISSN: 1525-3961, DOI: 10.1007/S10162-017-0634-8 [retrieved on 2017-08-16]

## Description

### BACKGROUND OF THE INVENTION

More than half of the cases of nonsyndromic profound congenital deafness have a genetic cause, and most (~ 80%) are autosomal recessive (DFNB) forms (Duman D. & Tekin M, Front Biosci (Landmark Ed) 17:2213-2236 (2012)). Genetic diagnosis of deafness provides essential information for cochlear gene therapies, and rapid progress has been made in both the accuracy and accessibility to genetic testing in the last few years. Identification of mutations in syndromic deafness genes could be many years before the emergence of symptoms in patients, giving time for planning disease management.

Deafness genes encode proteins with a wide range of molecular functions vital for cochlear functioning, such as development of the sensory organ, sound transduction in the stereocilia of hair cells, maintenance of the endocochlear potential (EP) and high concentration of extracellular potassium, and synaptic neurotransmission between hair cells and spiral ganglion neurons (SGNs). Major proteins made from deafness genes include ion channels and transporters, gap junctions and tight junctions, protein subunits in cytoskeleton and molecular motors, and transcription factors transiently expressed in cochlear development. Whether a mutation affects early cochlear development and leads to a significant cellular degeneration is a major factor in determining the "treatment time window", which is a crucial problem in this therapeutic field.

Prosthetic cochlear implants are currently used for rehabilitation (Kral A & O'Donoghue GM N Engl J Med 363(15):1438-1450 (2010)), but hearing recovery is far from perfect, particularly for the perception of speech in noisy environments or of music, because of their inherent limitation of frequency resolution as imposed by inter-channel electrical interference.

A primary motivation in developing biological treatments is to restore hearing without the implantation of any prosthetic device, and to achieve sound resolution quality and unit cost that is much better than what is currently achievable with cochlear implants. In particular, gene therapy with local Adeno-associated virus (AAV)-mediated gene therapy has already been proposed for treating human forms of deafness (Zhang et al, Frontiers in Molecular Neuroscience, vol.11, Art.221, 2018).

Using an Ush1c c.216G>A knock-in mouse model to study the Usher typeI C disease, Pan et al. Nature biotechnology; 35(3):264-272 (2017) tested whether cochlear gene therapy could be used to target hair cells to correct the deafness phenotype. A novel synthetic AAV, Anc80L65, was able to transduce >90% of hair cells. The treatment demonstrates morphological preservation in the cochlea, and the auditory thresholds were improved for 60-70 dB compared to untreated ears when recombinant viral vectors were injected at P0-P1 (i.e., the day the mice are born or the day after, that are called "post-natal days 0 or 1"). The same injections performed at P10-P12 (i.e., ten or twelves days after birth, just before the hearing onset - which occurs around P12 in this species) didn't yield any treatment effect (Pan et al. Nature biotechnology; 35(3):264-272 (2017).

Akil et al. Neuron 75:283-293 (2012) used AAV2/1 to deliver the vesicular glutamate transporter 3 (*Vglut3)* cDNA into neonatal (i.e., postnatal days 1 to 12) KO mouse cochleas in order to treat a disorder of synaptic transmission of the inner hair cells. Based on data from auditory brainstem response (ABR) and acoustic startle reflexes, they demonstrated that auditory function in injected ears recovered within 2 weeks.

These two studies confirm significant alleviation of cellular degeneration in the cochlea when recombinant viral vectors were injected in early postnatal stages, i.e., before maturation of the mouse hair cells.

To date, all gene therapy studies having evaluated the inner ear therapy in mice have concluded that these therapies are not efficient in adult animals. There is thus a consensus that there is a critical period for gene therapy to be effective in hearing preservation or recovery, and that a window of opportunity for treatment only exists in mice at embryonic or at early postnatal stage, i.e., before the hearing onset occurs (Ahmed et al, JARO 18:649-670 (2017).

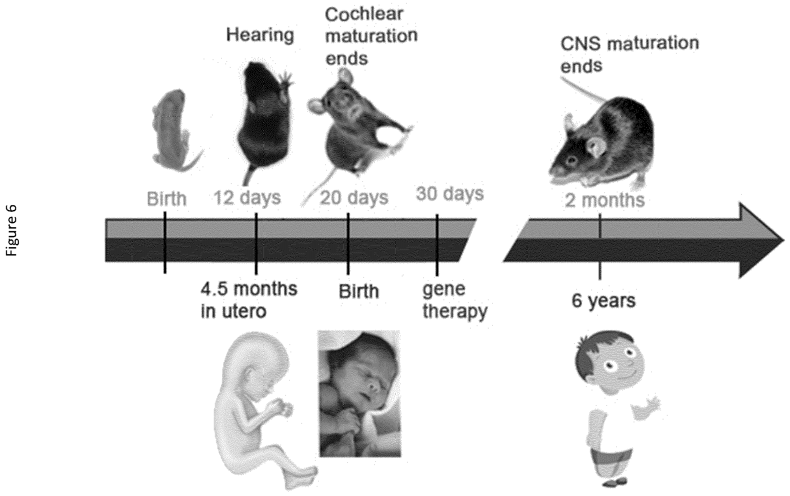

It is well-known that the mouse inner ear is still structurally and functionally immature at birth, and that the hearing onset takes place in this animal species at postnatal day 12 (P12) to end around postnatal day 20 (P20) (Shnerson and Willott, J. Comp. Physiol. Psychol. 94, 36-40 (1980)). However, the hearing onset and maturation occurs in a completely different timing in humans. As a matter of fact, the human inner ear is capable of auditory function as early as 4.5 month *in utero* and is ended at birth (cf. figure 6 and Hepper PG & Shahidullah BS Arch Dis Child71 (2):F81-87 (1994)).

This means that the experiments of the prior art, aiming at restoring the auditory functions before the onset of hearing or before the end of the cochlear maturation, should be performed in utero when transposed to human being trials (figure 6). Yet, no human being has ever been treated with hair cells gene therapy so far, because restoring the hearing in the postnatal stage or infancy (i.e., when the auditory system is entirely mature and functional), was thought impossible.

Nevertheless, the ultimate goal for cochlear gene therapy is the treatment of common genetic deafness in humans after a potential genetically induced deafness can be detected or diagnosed i.e., most of the time, after their birth.

It is indeed not possible to develop gene therapy protocols that involve *in utero* delivery of treatments into the cochlea, because this surgical operation is likely to induce a number of side effects, among which a definitive hearing loss (Zhang et al, Frontiers in Molecular Neuroscience, vol.11, Art.221, 2018). Moreover, it is well-known that DFNB deafness are typically diagnosed in humans during the neonatal period, i.e., after birth, between 0 and 4 months.

To be transposable to humans, gene therapy approaches should therefore be tested and efficient in reversing established deafness phenotype affecting mature auditory systems, for example when administered to mice at postnatal days > P20 (corresponding to young or adult humans).

This will be the only way to identify treatments whose time window is compatible with human ethics and welfare, because they can be administered in post-natal, infant and adult humans, in which the auditory system is completed.

### DETAILED DESCRIPTION OF THE INVENTION

The present inventors have developed alternative studies in order to identify treatments that have a realistic chance to efficiently prevent or reverse hearing loss in a subject, especially a human, in which the auditory system, especially the cochlea, is mature, yet without involving embryonic gene delivery. In this context, they have been able to demonstrate that the recombinant expression of the Otoferlin protein in inner hair cells is able to restore the audition in model mice treated at postnatal days > P20 (corresponding to young or adult humans).

Otoferlin is abundantly expressed in sensory inner hair cells (IHCs) of the cochlea. It is also expressed in other cells of the central nervous system. It plays a key role in the final steps of synaptic vesicle fusion at cochlear hair cell synapses with afferent spiral ganglion neurons. More precisely, it is important for exocytosis at the auditory ribbon synapse (Roux et al, Cell 127(2):277-89, 2006).

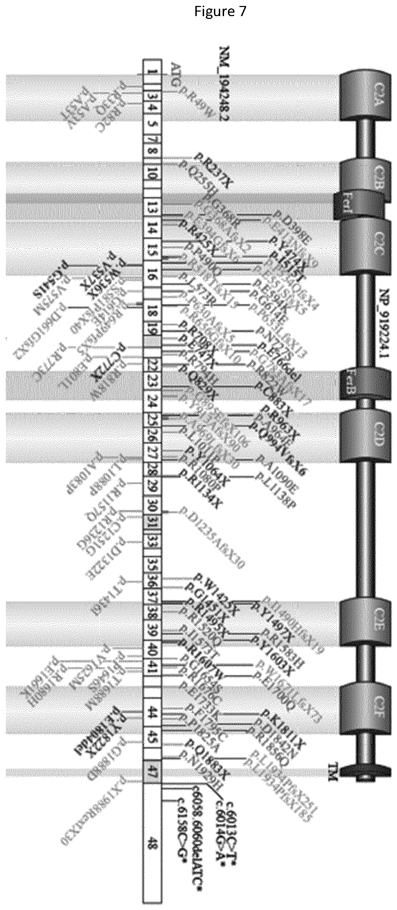

In human beings, mutations affecting the *Otoferlin* gene ("OTOF gene") lead to severe non-syndromic bilateral loss of hearing that occurs after birth but before the acquiring of language. Some of them also lead to a temperature-sensitive nonsyndromic auditory neuropathy, that is triggered when the body temperature increases importantly (for example in case of fever, see Marlin S. et al, Biochemical and Biophysical Research Communications, 394 (2010) 737-742 and Varga R. et al, J. Med. Genet 2006; 43:576-581). At least 60 mutations have been identified so far (cf. figure 7), among which 5 are known to be thermosensitive (P.Q994VfsX6, P.I515T, p.G541S, PR1607W, p.E1804del as described in Pangrsic T. et al, Trends in Neurosciences, 2012, col.35, No.11).

These two deafness phenotypes (constitutive and inducible) are found all over the world and known as the "Deafness, Autosomal Recessive 9" or "DFNB9" deafness.

DFNB9 deafness accounts for up to 8% of autosomal recessive non-syndromic hearing loss in some Western populations, thereby residing within the top five of genetic hearing disorders that still require a therapeutic intervention.

The present inventors report here, in the DFNB9 mouse model (OTOF knock-out mice), the first proof-of-principle that cochlear delivery of a fragmented cDNA via a dual-AAV vector approach can effectively and long-lastingly correct the profound deafness phenotype of these mice when administered well after their auditory system has matured (P30). This result suggests that the therapeutic window for local gene transfer in patients with congenital deafness due to DFNB9 is in fact longer than initially suspected.

As a matter of fact, the administration of the vectors of the invention, each providing a portion of the OTOF gene and enabling for the expression of the full-length OTOF protein in the transfected inner hair cells of OTOF knock-out mice at late postnatal days (P30), lead to better results than when younger mice were treated.

This is very surprising given that it was taught in a number of study that the therapeutic window for local gene transfer in mice affected by genetic deafness was embryonic or in early post-natal days (Ahmed et al, JARO 18:649-670 (2017); Zhang et al, Frontiers in Molecular Neuroscience, vol.11, Art.221, 2018).

It is known that, in mice, the full range of responsive frequencies can be observed by P14 (i.e., fourteen days after birth). Response latencies and interpeak intervals matured rapidly over the course of the second and third postnatal weeks and achieve adultlike characteristics at P18 (Song L. et al, J Acoust Soc Am 119(4):2242-2257 (2006)). At P30, the auditory system of the mice is therefore completely matured. It corresponds to the auditory system of an infant or adult human (see figure 6).

Hence, the results obtained by the inventors suggest that the gene therapy used in the present invention can be efficient in humans not only in a pre-natal time window, but also in infant patients that are diagnosed to suffer from congenital DFNB9 deafness, or in adult patients that are diagnosed later, for example because they carry thermosensitive mutations in the *OTOF* gene.

In a first aspect, the present invention relates to a vector system comprising at least two AAV particles, each of them comprising a polynucleotide comprising a partial coding sequence that encodes i) the N-terminal part of the Otoferlin polypeptide or of a functional fragment thereof, for one, and ii) the C-terminal part of the Otoferlin polypeptide or of a functional fragment thereof, for the other, said vector system allowing the expression of the full-length Otoferlin polypeptide, or of a functional fragment thereof, in inner hair cells, for use for treating patients suffering from DFNB9 deafness or preventing DFNB9 deafness in patients having DFNB9 mutations, wherein said patients are human having a developed and mature auditory system, wherein said patients are preferably toddlers, infants, teenagers or adult humans.

As used herein, the term "Otoferlin" designates the Otoferlin polypeptide. It is herein abbreviated as "OTOF". This polypeptide is also known as "AUNB1"; "DFNB6"; "DFNB9"; "NSRD9" and "FER1L2".

The full-length of the isoform 1 of the wild-type human Otoferlin polypeptide is presented in SEQ ID NO:1 (corresponding to Genbank number AF183185.1). This polypeptide is a member of the Ferlin family of transmembrane proteins, which has C2 domains as synaptotagmins, PKC and PLC. This long form contains six C2 domains. As mentioned above, it is involved in synaptic vesicle fusion between cochlear hair cell and afferent spiral ganglion neurons (Roux et al, Cell 127(2):277-89, 2006; Michalski et al, Elife, 2017 Nov 7;6 e31013).

The term "Otoferlin polypeptide" designates the Otoferlin polypeptide of SEQ ID NO:1 and homologous sequences thereof, that retain at least one biological function of the Otoferlin polypeptide that is of interest in the present context. For example, this biological function is related to the modulation of vesicles fusion at the cochlear inner hair cell ribbon synapses that activate the primary auditory neurons (Roux et al 2006; Michalski et al, Elife, 2017 Nov 7;6 e31013). This modulation could be assessed with classical *ex vivo* electrophysiological measures.

In a preferred embodiment, the vector system for use according to the invention allows for the expression of a homologous polypeptide whose amino acid sequence shares at least 70% identity and/or similarity with SEQ ID NO:1. Said homologous sequence more preferably shares at least 75%, and even more preferably at least 80%, or at least 90% identity and/or similarity with SEQ ID NO:1. When the homologous polypeptide is much shorter than SEQ ID NO:1, then local alignment can be considered.

Said homologous polypeptide can have for example the amino acid sequence presented in SEQ ID NO:5 (corresponding to Genbank number NP_001274418). Said sequence characterises the isoform e (variant 5) of the wild-type human Otoferlin polypeptide. This isoform e is encoded by the cDNA variant having the sequence SEQ ID NO:22. This variant lacks an alternate in-frame exon in the 3' coding region and uses a downstream stop codon compared to SEQ ID NO:1. It encodes a distinct C-terminus as compared to SEQ ID NO:1 (but its N-terminal part is the same).

Said homologous polypeptide can also have the amino acid sequence presented in SEQ ID NO:6 (corresponding to Genbank number NP_004793.2) or the amino acid presented in SEQ ID NO:24 (corresponding to Genbank number NP_919303.1) corresponding to the short isoforms b and c (variants 2 and 3) respectively. More precisely, SEQ ID NO:6 represents the isoform b (variant 2, also called 'short form 1') which has a shorter N-terminus and lacks a segment compared to SEQ ID NO:1. On another hand, SEQ ID NO:24 represents the isoform c (variant 3, also called "short form 2"), which differs in the 5' UTR and coding sequence compared to variant 1 (SEQ ID NO:1) because it has a shorter and distinct C-terminus compared to SEQ ID NO:1.

Said homologous sequence can also be for example the Otoferlin polypeptide of another animal species, such as SEQ ID NO:7 which is the mouse full-length isoform 1 of the Otoferlin polypeptide (corresponding to Genbank number NP_001093865.1). This isoform is encoded by the cDNA of SEQ ID NO:16 (NM_1100395).

In the context of the invention, when the identity percentage between said two homologous sequences can be identified by a global alignment of the sequences in their entirety (e.g., when the sequences are of about the same size), this alignment can be performed by means of an algorithm that is well known by the skilled person, such as the one disclosed in Needleman and Wunsch (1970). Accordingly, sequence comparisons between two amino acid sequences or two nucleotide sequences can be performed for example by using any software known by the skilled person, such as the "needle" software using the "Gap open" parameter of 10, the "Gap extend" parameter of 0.5 and the "Blosum 62" matrix.

When local alignment of the sequences is to be considered (e.g., in case of homologs that have a smaller size than the sequences of the invention), then said alignment can be performed by means of a conventional algorithm such as the one disclosed in Smith and Waterman (J. Mol. Evol. 1981; 18(1) 38-46).

The invention provides systems encoding homologous amino acid sequences that are "similar" to SEQ ID NO:1 or SEQ ID NO:5 or SEQ ID NO: 6 or SEQ ID NO:24. "Similarity" of two targeted amino acid sequences can be determined by calculating a similarity score for the two amino acid sequences. As used herein, the "similarity score" refers to the score generated for the two sequences using the BLOSUM62 amino acid substitution matrix, a gap existence penalty of 11, and a gap extension penalty of 1, when the two sequences are optimally aligned. Two sequences are "optimally aligned" when they are aligned so as to produce the maximum possible score for that pair of sequences, which might require the introduction of gaps in one or both of the sequences to achieve that maximum score. Two amino acid sequences are substantially similar if their similarity score exceeds a certain threshold value. The threshold value can be any integer ranging from at least 1190 to the highest possible score for a particular reference sequence (e.g., SEQ ID NO:1). For example, the threshold similarity score can be 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, or higher. If in a particular embodiment of the invention, the threshold score is set at, for example, 1300, and the reference sequence is SEQ ID NO:1, then any amino acid sequence that can be optimally aligned with SEQ ID NO:1 to generate a similarity score of greater than 1300 is "similar" to SEQ ID NO:1.

Amino acid substitution matrices and their use in quantifying the similarity between two sequences are well-known in the art and described, e.g., in Dayhoff et al. (1978), "A model of evolutionary change in proteins", "Atlas of Protein Sequence and Structure," Vol. 5, Suppl. 3 (ed. M. O. Dayhoff), pp. 345-352. Natl. Biomed. Res. Found., Washington, D.C. and in Henikoff et al. (1992) Proc. Natl. Acad. Sci. USA 89:10915-10919. While optimal alignment and scoring can be accomplished manually, the process is facilitated by the use of a computer-implemented alignment algorithm, e.g., gapped BLAST 2.0, described in Altschul et al., (1997) Nucleic Acids Res. 25:3389-3402, and made available to the public at the National Center for Biotechnology Information website. To generate accurate similarity scores using NCBI BLAST, it is important to turn off any filtering, e.g., low complexity filtering, and to disable the use of composition based statistics. One should also confirm that the correct substitution matrix and gap penalties are used. Optimal alignments, including multiple alignments, can be prepared using, e.g., PSI-BLAST, available through the NCBI internet site and described by Altschul et al., (1997) Nucleic Acids Res. 25:3389-3402.

In another embodiment, the vector system for use according to the invention can allow for the expression of a functional fragment of the Otoferlin polypeptide. The term "**functional fragment**" herein designates any fragment of the human Otoferlin polypeptide or any fragment of a polypeptide having a homologous sequence as defined above, wherein said fragment retains at least one biological function of the Otoferlin polypeptide that is of interest in the present context. For example, this biological function is related to the modulation of vesicles fusion at the cochlear inner hair cell ribbon synapses that activate the primary auditory neurons (Roux et al 2006; Michalski et al, Elife, 2017 Nov 7;6 e31013). This modulation could be assessed with classical ex *vivo* electrophysiological measures.

For example, said functional fragment can have the amino acid sequence presented in SEQ ID NO:6 (corresponding to isoform b having a Genbank number NP_004793.2) or in SEQ ID NO:24 (corresponding to isoform c having a Genbank number NP_919303.1). Said sequences characterise short isoforms of the wild-type human Otoferlin polypeptide, comprising only three C2 domains.

In this aspect of the invention, the vector system for use according to the invention is administered to patients suffering from DFNB9 deafness. By "patients suffering from DFNB9 deafness", it is herein meant a patient, especially a human patient, that is thought to have (or has been diagnosed to have) a mutation in the constitutive Otoferlin gene, said mutation triggering an abnormal expression, function or both, of the Otoferlin protein. In a particular embodiment, said mutation can be thermo-sensitive.

To date, more than 60 pathogenic mutations have been reported in otoferlin (see Figure 7). Among these are at least five thermosensitive mutations, identified in patients affected by episodic deafness conditioned by fever (P.Q994VfsX6, P.I515T, p.G541S, PR1607W, p.E1804del).

These patients can be identified by the skilled physician, e.g., using a combination of electrophysiologic testing of auditory brain stem responses (ABRs) and/or genetic testing to identify mutations in the OTOF gene. In some embodiments, the patient has one or more of the following nonsense or missense mutations in the OTOF gene: TYR730TER, GLN829TER, PR01825ALA, PRO50ARG, LEU1011PRO, ILE515THR, ARG1939GLN,or GLY541SER. In some embodiments, the patient has an A-to-G transition at the intron 8/exon 9 junction (IVS8-2AG) or a G-to-A transition at position +1, the first intronic nucleotide in the splice donor site of exon 5 or a G-C transversion in the donor splice site of intron 39. In some embodiments, the patient has a one base pair deletion (1778G) in exon 16, leading to a stop codon, and a 6141G-A change, resulting in an ARG-to-GLN substitution in exon 48.

The patients to which the vector system for use according to the invention is administered are patients, especially human patients, in which the auditory system, especially the cochlea, is already developed and mature. These patients, especially human patients, are therefore not human embryos or foetuses, because the administration is not intended to be performed *in utero.* According to figure 7, the patients targeted by the present invention are preferably new born human babies, typically younger than 6 months old, or even younger than 3 months old, if DFNB9 deafness is diagnosed that young. These human babies are more preferably between 3 months and 1 year.

Of note the human cochlea as a whole attains an adult size between 17 and 19 weeks' gestation and is fully morphologically mature at 30-36 weeks (corresponding to 12 days after birth in the mouse). The functional maturation of the inner hair cell ribbon synapse can be evaluated by monitoring the wave I of the ABR recording, that can be recorded at about the 28th week of gestation in humans. Recordings and analyses of the ABR wave I (reflecting the function of the inner hair cell synapses with the primary auditory neurons) have shown a complete functional maturation in human babies at birth (corresponding to 20 days after birth in the mouse). This is well known in the art (see for example Pujol et Lavigne-Rebillard, Acta oto-laryngologica. Supplementum · February 1991).

It is therefore also possible to administer the vector system for use according to the invention to older human patients, such as toddlers (2-6 year old), infants (6-12 year old), teenagers (12-18 year old) or adult humans (18 years and over).

Altogether, it is therefore preferred to administer the vector system for use according to the invention to human patients aged between 3 months and 25 years.

The patients of the invention are in particular human infants diagnosed as being affected by DFNB9 deafness after language acquisition.

In another particular embodiment, the patients are human beings that are 6 years and older, i.e., the administration of the treatment occurs when their Central Nervous System is completely mature (cf. figure 7).

In particular embodiment, the vector system for use according to the invention is administered to human patients suffering from DFNB9 deafness induced by thermosensitive mutations, preferably to teenagers or adult humans carrying at least one of the Otoferline thermosensitive mutations mentioned above.

As used herein, the term "**treating**" is intended to mean the administration of a therapeutically effective amount of one of the vector system of the invention to a patient suffering from DFNB9 deafness, in order to restore partially or completely the hearing in said patient. Said recovery can be assessed by testing the auditory brain stem responses (ABRs) with electrophysiologic devices. "Treatment of the DFNB9 deafness" is in particular intended to designate the complete restoration of hearing function regardless of the cellular mechanisms involved.

For patients carrying thermo-sensitive mutations, the vector system can also be administered to prevent the loss of hearing induced from the body temperature modulation. In the context of the present invention, the term "**preventing**" designates impairing or delaying the loss of hearing within audible frequency range.

In these and other DFNB9 patients, the vector system can be administered both for preventing the loss of hearing before it occurs, and for restoring the hearing capacity when hearing loss has already occurred.

Multiple routes of delivery to the inner ear have been explored. These include injection into the perilymphatic spaces via the round window membrane (RWM) and via the oval window and injection into the scala tympani or scala vestibule via cochleostomy. Distribution throughout the perilymphatic spaces has been demonstrated for all these routes of delivery. Furthermore, it has been demonstrated that advection flow through the cochlea and vestibular organs can facilitate distribution of therapeutic agents from the site of injection to more distant regions of the inner ear. Delivery into the endolymphatic spaces has also been explored via cochleostomy into the scala media, via canalostomy and by injection into the endolymphatic sac. These approaches have also yielded broad distribution but face the added challenge of breaching the barrier between high potassium endolymph and perilymph. Disruption of the barrier poses two potential problems. First, leakage of high potassium into the perilymphatic spaces that bathe the basolateral surface of hair cells and neurons can chronically depolarize these cells and lead to cell death. Second, breach of the tight junctions between endolymph and perilymph can lead to rundown of the endocochlear potential which typically ranges between +80 and +120 mV. Rundown of the endocochlear potential reduces the driving force for sensory transduction in hair cells and therefore leads to reduced cochlear sensitivity and elevated auditory thresholds. Avoiding these complications is particularly challenging in the adult cochlea. However, by targeting endolymphatic spaces in the vestibular system, which does not have an endolymphatic potential, but are continuous with cochlear endolymph spaces, these confounding issues may be minimized while still providing sufficient distribution within the cochlea (Ahmed et al, JARO 18:649-670 (2017)).

The cochlea is highly compartmentalized and separated from the rest of the body by the blood-cochlear barrier (BCB), which minimizes the therapeutic injection volume and leakage into the body's general circulation system, to protect cochlear immune privilege and reduce the chance of systemic adverse immune responses. As the hair cells and supporting cells in the cochlea normally do not divide, the cells in the cochlea remain stable, therefore making it possible to use non integrating viral vectors (e.g., AAV) for sustained transgene expression.

The semi-circular approach has been suggested as a promising injection route for future cochlear gene therapy in human trials since the posterior semi-circular canal also appears to be accessible in humans (Suzuki et al., Sci. Rep. 7:45524 (2017); Yoshimura et al., Sci. Rep. 8:2980 (2018).

In a preferred embodiment of the invention, the vector system is administered in human ear via one of the two common and well-established techniques that are routinely used in clinical otologic surgical practice. More precisely, these approaches will be adopted to target the perilymphatic spaces. To this end, the injections using a micro-catheter will be carried out either through the oval window using laser stapedotomy (trans-stapes) or transmastoid / trans-round window (Dai C. et al, JARO, 18:601-617, 2017).

Systemic administration by intravenous injections or infusions are also possible.

The vector system for use according to contains at least one polynucleotide vector that can trigger the expression of the full-length Otoferlin polypeptide, or a functional fragment thereof, in inner hair cells. Preferably, said polynucleotide vector contains a coding sequence encoding said polypeptide, or a functional fragment thereof, which is operatively linked with a promoter that enables the expression of the gene in said cells specifically.

Said coding sequence is for example the human *Otoferlin* gene of SEQ ID NO: 2, corresponding to the cDNA sequence of the isoform 1 of the human wild-type Otoferlin gene (NM_194248.2 = isoform a or variant 1, which is the longest isoform).

Said coding sequence can also be the shorter cDNA sequence NM_001287489.1 (isoform e or variant 5) of SEQ ID NO:22, the cDNA sequence NM_004802.3 (isoform b or variant 2), the cDNA sequence NM_194322.2 (isoform c or variant 3), or the cDNA sequence NM_194323.2 (isoform d or variant 4).

A number of viral and nonviral vectors have been developed for delivery of genetic material in various tissues and organs. In most cases, these vectors are replication incompetent and pose little threat of viral-induced disease. Rather, the viral genome has been partly or fully deleted, expanding the capacity to allow inclusion of therapeutic DNA cargo within the viral capsid. Some vectors include single-stranded DNA, while others include double-stranded DNA. Particularly preferred vectors in the context of the invention are lentiviral vectors, adenovirus vectors, Adeno-associated viruses (AAV) as disclosed in Ahmed et al, JARO 18:649-670 (2017).

Specifically, AAVs are small replication-deficient adenovirus-dependent viruses from the *Parvoviridae* family. They have an icosaedrical capsid of 20-25 nm in diameter and a genome of 4.8 kb flanked by two inverted terminal repeats (ITRs). After uncoating in a host cell, the AAV genome can persist in a stable episome state by forming high molecular weight head-to-tail circular concatamers, or can integrate into the host cell genome. Both scenarios provide long-term and high-level transgene expression.

AAV appears to be a promising virus for cochlear gene therapies based on results obtained in human trials of ocular gene therapy. The reasons for the success of AAV in human ocular gene therapy include: (1) proven safety profile (large number of human trials have shown that AAV lack pathogenicity and possess very low immunogenicity), (2) long-lasting transgene expression in non-dividing cells, (3) the small size of AAV (≈20 nm, which is five times smaller than Adenoviruses) helps the diffusion across cellular barriers to reach targeted cells (Zhang et al, Frontiers in Molecular Neuroscience, vol.11, Art.221 , 2018).

The vector system for use according to the invention comprises at least two AAV particles, each of them comprising a polynucleotide comprising a partial coding sequence that encodes i) the N-terminal part of the Otoferlin polypeptide, or of a functional fragment thereof, for one, and ii) the C-terminal part of the Otoferlin polypeptide, or of a functional fragment thereof, for the other.

Twelve natural occurring serotypes of human AAV have been characterized to date. These serotypes have different inherent tropisms and transduction efficiencies in muscles, lung, liver, brain, retina, and vasculature. Multiple attempts of AAV pseudotyping and capsid engineering resulted in considerable improvement of tropism and efficiency of transduction. As for cells of the inner ear, AAV1-4, 7, and 8 were shown to infect spiral limbus, spiral ligament, and spiral ganglion cells in vivo. Infection of IHCs was also shown for AAV1-3, 5, 6, and 8. AAV1 was the most effective and occasionally infected OHCs and supporting cells. Also, AAV5 was shown to be efficient for Claudius cells, spiral ganglion, and inner sulcus cells. Among pseudotyped vectors, AAV2/1 was found to efficiently transduce progenitor cells giving rise to IHCs and OHCs in mouse cochlea, and AAV2/2 was optimal for IHCs of guinea pig cochlea (Ahmed et al, JARO 18:649-670 (2017)).

Thus, in a preferred embodiment, the vector system contains an AAV vector chosen in the group consisting of: AAV1, AAV2, AAV3, AAV4, AAV5, AAV6, AAV7, AAV8, AAV9, and AAV10.

In a more preferred embodiment, the serotype of said vector is AAV2, AAV8, AAV5, or AAV1. In an even more preferred embodiment, the serotype of said vector is AAV2 or AAV8. AAV8, which is the most preferred serotype in the context of the present invention, is currently tested *in vivo.*

In order to increase the efficacy of gene expression, and prevent the unintended spread of the virus, genetic modifications of AAV can be performed. These genetic modifications include the deletion of the E1 region, deletion of the E1 region along with deletion of either the E2 or E4 region, or deletion of the entire adenovirus genome except the cis-acting inverted terminal repeats and a packaging signal. Such vectors are advantageously encompassed in the present invention.

Moreover, genetically modified AAV having a mutated capsid protein may be used so as to direct the gene expression towards a particular tissue type, e.g., to auditory cells. In this aim, modified serotype-2 and -8 AAV vectors in which tyrosine residues in the viral envelope are substituted for alanine residues can be used. In the case of tyrosine mutant serotype-2, tyrosine 444 can be substituted with alanine (AAV2-Y444A). In the case of serotype 8, tyrosine 733 can be substituted with an alanine reside (AAV8-Y733A). By using AAV2-Y444A or AAV8-Y733A, it is possible to increase gene transfer by up to 10,000 fold, decreasing the amount of AAV necessary to infect the sensory hair cells of the cochlea.

In a preferred embodiment, the polynucleotide(s) expressing the *Otoferlin* polypeptide or gene or functional fragment thereof, is contained in recombinant AAV2 particles in which all the tyrosine residues have been replaced by phenylalanine residues (AAV2 (Y->F) or Quad Y-F, as disclosed in Petrs-Silva H et al, Mol. Ther. 19, 293-301 (2011) and in the examples below. Mutated tyrosine residues on the outer surface of the capsid proteins include, for example, but are not limited to, mutations of Tyr252 to Phe252 (Y252F), Tyr272 to Phe272 (Y272F), Tyr444 to Phe444 (Y444F), Tyr500 to Phe500 (Y500F), Tyr700 to Phe700 (Y700F), Tyr704 to Phe704 (Y704F), Tyr730 to Phe730 (Y730F) and Tyr733 to Phe733 (Y733F). These modified vectors facilitate penetration of the vector across the round window membranes, which allow for non-invasive delivery of the vectors to the hair cells/spiral ganglion neurons of the cochlea. These mutated vectors avoid degradation by the proteasome, and their transduction efficiency is significantly increased.

Other recombinant AAV particles that derivate from the natural serotypes 1-10 include AAV2-AAV3 hybrids, AAVrh.10, AAVhu.14, AAV3a/3b, AAVrh32.33, AAV-HSC15, AAV-HSC17, AAVhu.37, AAVrh.8, CHt-P6, AAV2.5, AAV6.2, AAV2i8, AAV-HSC15/17, AAVM41, AAV9.45, AAV6 (Y445F/Y731F), AAV2.5T, AAV-HAE1/2, AAV clone 32/83, AAVShH10, AAV8 (Y733F), AAV2.15, AAV2.4, AAVM41, and AAVr3.45 (Asokan A. et al, Mol. Therapy, vol.20 n°4, 699-708, 2012).

It is also possible to use the synthetic vector Anc80L65 which has been shown to have the highest efficiency for transduction of inner ear hair cells reported to date (Suzuki et al., Sci. Rep. 7:45524 (2017)). It is also possible to use exosome-associated AAVs as proposed by György et al, Mol. Ther. 25(2):379-391, 2017.

It is also possible to use overloaded AAV/PHP.B vectors whose capsids have been modified so as to enhance their packaging capacity.

Methods for preparing viruses and virions comprising a heterologous polynucleotide or construct are known in the art. In the case of AAV, cells can be coinfected or transfected with adenovirus or polynucleotide constructs comprising adenovirus genes suitable for AAV helper function. Examples of materials and methods are described, for example, in U.S. 8,137,962 and 6,967,018.

The skilled person would easily determine if it is required, prior to the administration of the vector(s) of the invention, to enhance the permeability of the round window membrane as proposed in WO 2011/075838, depending on the target cell.

Even when AAV is used, the system can be a one-vector system. In this case modified capsids may be used (cf. AAV/PHP.B vectors).

If the chosen AAV capsid has a limited packaging capacity of 5 kilobases, it is better to use dual vector systems as disclosed for example in WO 2013/075008, which is incorporated herein by reference.

The present inventors have used said dual-AAV vector approach to provide the two half-portions of the *Otoferlin* gene to inner hair cells, where an homologous recombination occurs and the full-length protein is expressed. Their results show that the two distinct AAVs vectors are able to transduce efficiently the targeted inner hair cells, where the Otoferlin protein is produced and restores in a long-lasting way the profound deafness phenotype of mice OTOF KO that suffer from congenital deafness due to DFNB9 invalidation.

Accordingly, the vector system for use according to the invention preferably comprises at least two AAV particles, each of said AAV particles comprising either:
a) a first polynucleotide comprising an inverted terminal repeat at each end of said polynucleotide, and, between the said inverted terminal repeats, from 5' to 3': a suitable promoter followed by a partial coding sequence that contains the N-terminal part of the *Otoferlin* gene, and a splice donor site, or
b) a second polynucleotide comprising an inverted terminal repeat at each end of said polynucleotide, and, between the said inverted terminal repeats, from 5' to 3': a splice acceptor site, a partial coding sequence that contains the C-terminal part of the *Otoferlin* gene, optionally followed by a polyadenylation sequence,

wherein the said first and second polynucleotides also contain a recombinogenic sequence that is located after the splice donor site in said first polynucleotide and before the splice acceptor site in said second polynucleotide, and
wherein the coding sequences in the first and second polynucleotides when combined encode the full-length of the Otoferlin polypeptide.

This preferred embodiment uses a "first" and a "second" polynucleotide. It is however understood that "first" and "second" are not meant to imply a particular order or importance, unless expressly stated otherwise.

As explains in figure 1 and in WO 2013/075008, the first and second polynucleotides used in this particular embodiment should contain specific genetic components in order to induce the appropriate recombination and expression of the Otoferlin protein in the target cells.

These specific components are the following:

### • ITRs

In some embodiment of the invention, the partial or the full-length cDNA of the *OTOF* gene is inserted into two ITR-containing plasmids.

If AAV vector(s) is used, the ITR sequences of a polynucleotide described herein can be derived from any AAV serotype (e.g., 1, 2, 3, 4, 5, 6, 7, 8, 9, or 10) or can be derived from more than one serotype. In some embodiments of the polynucleotide provided herein, the ITR sequences are derived from AAV2. ITR sequences and plasmids containing ITR sequences are known in the art and commercially available.

An exemplary AAV2 ITR sequence for flanking the 5' end of an expression construct comprises the sequence SEQ ID NO:10. An exemplary AAV2 ITR sequence for flanking the 3' end of an expression construct comprises the sequence SEQ ID NO:11.

Such ITRs can also advantageously be used if the polynucleotide is a single vector system.

### • A suitable promoter

Promoters contemplated for use in the vector system include, but are not limited to, cytomegalovirus (CMV) promoter, SV40 promoter, Rous sarcoma virus (RSV) promoter, chimeric CMV/chicken beta-actin promoter (CBA) and the truncated form of CBA (smCBA) (U.S. Patent No. 8,298,818). In a specific embodiment, the promoter is a chimeric promoter comprising CMV and beta-actin promoter.

In a preferred embodiment, the promoter used in the vector system for use according to the invention is the truncated chimeric CMV β actin (smcBA) promoter of SEQ ID NO:8 or a promoter comprising a sequence identity of at least 75%, at least 80%, at least 85%, at least 90%, at least 95%, at least 96%, at least 97%, at least 98%, at least 99% or 100% with SEQ ID NO:8.

Such promoters can also advantageously be used if the polynucleotide of the invention is a single vector system.

Promoters can be incorporated into a vector using standard techniques known in the art. Multiple copies of promoters or multiple promoters can be used in the vector systems. In one embodiment, the promoter can be positioned about the same distance from the transcription start site as it is from the transcription start site in its natural genetic environment. Some variation in this distance is permitted without substantial decrease in promoter activity. A transcription start site is typically included in the vector.
- A recombinogenic sequence that promotes the homologous recombination of the two half-sequences of the cDNA *in vivo,* so as to produce the entire coding sequence of the *OTOF* polypeptide and expression thereof in the transfected inner hair cells.

In some embodiments, the two polynucleotides (e.g., first and second polynucleotides) comprise a so-called "recombinogenic region" which can promote homologous recombination between the two polynucleotides once delivered to a cell (see, e.g., Ghosh et al. Hum Gene Ther. 2011 Jan;22(I):77-83).

This recombinogenic region typically consists in a first region of the first polynucleotide that has a homologous region in the second polynucleotide, or vice versa. The two regions preferably have a threshold level of sequence identity with each other of at least 75%, at least 80%, at least 90%, at least 95%, at least 96%, at least 97%, at least 98%, at least 99% or 100% identity, as defined above.

This recombinogenic region has preferably a size comprised between 50 and 500, 50 and 400, 50 and 300, 100 and 500, 100 and 400, 100 and 300, 200 and 500, 200 and 400, or 200 and 300 nucleotides.

In a preferred embodiment, the two regions are identical and have a size comprised between 200 and 300 nucleotides.

These recombinogenic sequences have sequences which are sufficiently homologous so as to permit hybridization with each other under standard stringent conditions and standard methods.

As used herein, "stringent" conditions for hybridization refers to conditions wherein hybridization is typically carried out overnight at 20-25°C below the melting temperature (Tm) of the DNA hybrid in 6x SSPE, 5x Denhardt's solution, 0.1% SDS, 0.1 mg/ml denatured DNA. The melting temperature is described by the following formula: Tm=81.5 C+16.6 Log[Na+]+0.41 (%G+C)-0.61 (% formamide)-600/length of duplex in base pairs.

Washes are typically earned out as follows: (1) Twice at room temperature for 15 minutes in 1x SSPE, 0.1 % SDS (low stringency wash). 2) Once at Tm-20°C for 15 minutes in 0.2x SSPE, 0.1% SDS (moderate stringency wash).

In a more preferred embodiment, the two regions are identical and have the sequence SEQ ID NO:9 or an homologous sequence having a sequence identity of at least 75%, at least 80%, at least 85%, at least 90%, at least 95%, at least 96%, at least 97%, at least 98%, at least 99% or 100% with SEQ ID NO:9.
- a splice donor site and a splice acceptor site

Once recombined in vivo, the full-length cDNA contains a splice donor / splice acceptor pair that causes splicing out of the recombinogenic region. The polynucleotides included in the dual-vector system comprise a splice donor or a splice acceptor site. In a preferred embodiment, the splice donor and/or splice acceptor sites contain splice consensus sequences. In a more preferred embodiment, the splice donor and/or splice acceptor sites carried by the polynucleotides included in the vector system contain splice consensus sequences derived from the alkaline phosphatase enzyme.

In a preferred embodiment, the polynucleotides included in the dual-vector system for use according to the invention comprise SEQ ID NO:12 and/or SEQ ID NO:13 as splice donor and acceptor site respectively, or splice sites comprising a sequence identity of at least 75%, at least 80%, at least 85%, at least 90%, at least 95%, at least 96%, at least 97%, at least 98%, at least 99% or 100% with SEQ ID NO:12 and/or SEQ ID NO:13.

The polynucleotide sequences, either contained in a single or in a dual vector system, may contain other regulatory components that are functional in the inner hair cells in which the vector is to be expressed. A person of ordinary skill in the art can select regulatory elements for use in human inner hair cells. Regulatory elements include, for example, internal ribosome entry site (IRES), transcription termination sequences, translation termination sequences, enhancers, and polyadenylation elements.

Transcription termination regions can typically be obtained from the 3' untranslated region of a eukaryotic or viral gene sequence. Transcription termination sequences can be positioned downstream of a coding sequence to provide for efficient termination. Signal peptide sequence is an amino terminal sequence that encodes information responsible for the relocation of an operably linked polypeptide to a wide range of post-translational cellular destinations, ranging from a specific organelle compartment to sites of protein action and the extracellular environment. Enhancers are cis-acting elements that increase gene transcription and can also be included in a vector. Enhancer elements are known in the art, and include, but are not limited to, the CaMV 35S enhancer element, cytomegalovirus (CMV) early promoter enhancer element, and the SV40 enhancer element.

DNA sequences which direct polyadenylation of the mRNA encoded by the structural gene can also be included in a vector.

A dual-vector approach is advantageous to split the coding sequence of the OTOF gene into two parts, in order to be packaged more easily into virions having a limited packaging capacity. When AAVs capsids are used, it is preferred to use polynucleotides that contain an OTOF coding sequence that contains no more than 5 kilobases, no more than 4 kilobases, and even more preferably no more than 3 kilobases.

The coding sequence of the human *OTOF* gene is preferably cut at a natural splicing site.

For example, the human *OTOF* gene isoform 1 of SEQ ID NO:2 can be split into a N-terminal part having a nucleotide sequence as presented in SEQ ID NO:3 (nucleotides 1-2676 of SEQ ID NO:2) and a C-terminal part having a nucleotide sequence as presented in SEQ ID NO:4 (nucleotides 2677-5994 of SEQ ID NO:2). And the human OTOF gene isoform 5 having SEQ ID NO:22 can be split into a N-terminal part of SEQ ID NO:3 and a C-terminal part of SEQ ID NO:23.

Exemplary polynucleotides that can be used as first and second polynucleotide in the vector system are for example SEQ ID NO:19 and SEQ ID NO:20 respectively. These two polynucleotides encode respectively the N-terminal and the C-terminal part of the isoform 1 of the Otoferlin human protein.

SEQ ID NO:19 contains:
- the 5 ITR sequence of AAV2 having the sequence SEQ ID NO: 10 (nt 20-162)
- the CMV enhancer (nt 186-440), the chicken β-actin promoter (nt 441 - 835), the exon 1 and chimeric intron of the β-actin protein (nt 836 - 1130), all the three being called "smCBA", corresponding to sequence SEQ ID NO:8,
- the 5' part of the human Otoferlin isoform 1 coding sequence having the sequence SEQ ID NO:3 (nt 1153-3558),
- the splice donor site of the Alkaline phosphatase having the sequence SEQ ID NO:12 (nt 3559-3642),
- the recombinogenic sequence having the sequence SEQ ID NO:9 (nt 3649-3935), and
- the 3' ITR sequence of AAV2 having the sequence SEQ ID NO:11.

On another hand, SEQ ID NO:20 contains:
- the 5' ITR sequence of AAV2 having the sequence SEQ ID NO: 10 (nt 20-162),
- the recombinogenic sequence having the sequence SEQ ID NO:9 (nt 207-493),
- the splice acceptor site of the Alkaline phosphatase having the sequence SEQ ID NO: 13 (nt 516-564),
- the 3' part of the human Otoferlin isoform 1 coding sequence having the sequence SEQ ID NO:4 (nt 565-4152),
   and
- the bovine growth hormone polyadenylation signal (nt 4190-4411), and
- the 3' ITR sequence of AAV2 having the sequence SEQ ID NO:11.

Exemplary polynucleotides that can be used as first and second polynucleotide in the vector system are for example SEQ ID NO:19 and SEQ ID NO:21 respectively. These two polynucleotides encode respectively the N-terminal and the C-terminal part of the isoform 5 of the Otoferlin human gene of SEQ ID NO:22 (as the N-terminal parts of isoforms 1 & 5 are identical, it is possible to use SEQ ID NO:19 for inducing the expression of the two isoforms).

SEQ ID NO:21 contains:
- the 5' ITR sequence of AAV2 having the sequence SEQ ID NO: 10 (nt 20-162),
- the recombinogenic sequence having the sequence SEQ ID NO:9 (nt 207-493),
- the splice acceptor site of the Alkaline phosphatase having the sequence SEQ ID NO: 13 (nt 516-564),
- the 3' part of the human Otoferlin isoform 5 coding sequence having the SEQ ID NO:23 (nt 565-4152),
   and
- the bovine growth hormone polyadenylation signal (nt 4190-4411), and
- the 3' ITR sequence of AAV2 having the sequence SEQ ID NO:11.

### Pharmaceutical compositions for use according to the invention

In another aspect, the present invention targets a pharmaceutical composition comprising the vector system as described above (i.e., the polynucleotides or the virions containing same), for use in treating patients, especially human patients, having a mature auditory

system, suffering from DFNB9 deafness or for preventing DFNB9 deafness in patients having DFNB9 mutations.

More generally, this pharmaceutical composition can be administered to human subjects suffering from congenital hearing loss due to altered *DFNB59* gene expression or deficiency. Said deficiency can be observed for example when Otoferlin is expressed at normal levels but is not functional.

Described is the use of the vector system as described above, for manufacturing pharmaceutical compositions intended to prevent and / or treat patients having a mature auditory system, especially human beings, suffering from the above-cited disorders, linked to altered *DFNB59* gene expression or deficiency.

As used herein, "**pharmaceutically acceptable carrier**" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it can be preferable to include isotonic agents, for example, sugars, polyalcohol such as mannitol, sorbitol, or sodium chloride in the composition. Pharmaceutically acceptable carriers can further comprise minor amounts of auxiliary substances such as wetting or emulsifying agents, preservatives or buffers, which enhance the shelf life or effectiveness of the antioxidant compounds or of the pharmaceutical compositions containing same.

The pharmaceutical compositions for use according to invention may be in a variety of forms. These include, for example, liquid, semi-solid and solid dosage forms, such as liquid solutions (e.g., injectable and infusible solutions), dispersions or suspensions, tablets, pills, powders, liposomes and suppositories. The form used depends on the intended mode of administration and therapeutic application. Typical compositions are in the form of injectable or infusible solutions.

Pharmaceutical compositions typically must be sterile and stable under the conditions of manufacture and storage. The pharmaceutical composition is preferably formulated as a solution, microemulsion, dispersion, liposome, or other ordered structure suitable to high drug concentration. Sterile injectable solutions can be prepared by incorporating the vectors in the required amount in an appropriate solvent with one or a combination of ingredients enumerated above, as required, followed by filtered sterilization. Generally, dispersions are prepared by incorporating the vectors into a sterile vehicle that contains a basic dispersion medium and the required other ingredients from those enumerated above. In the case of sterile lyophilized powders for the preparation of sterile injectable solutions, the preferred methods of preparation are vacuum drying and spray-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be achieved by including an agent in the composition that delays absorption, for example, monostearate salts and gelatin.

In the context of the invention, the typical mode of administration of the composition is intratympanic (in the middle ear), intracochlear, or parenteral (e.g., intravenous, subcutaneous, intraperitoneal, intramuscular, intrathecal). In one example, the pharmaceutical composition is administered by intravenous infusion or injection. In another example, the pharmaceutical composition of the invention is delivered to a specific location using stereostatic delivery, particularly through the tympanic membrane or mastoid into the middle ear.

More precisely, the compositions can be administered by using a micro-catheter that will be carried out either through the oval window using laser stapedotomy (trans-stapes) or transmastoid / trans-round window (Dai C. et al, JARO, 18:601-617, 2017).

In a preferred embodiment, the pharmaceutical composition of the invention is administered in human ear via intra-cochlear administration, more precisely by targeting endolymphatic spaces in the vestibular system or by the semi-circular approach mentioned above.

The pharmaceutical compositions for use according to the invention typically include a "**therapeutically effective amount**" or a "**prophylactically effective amount**" of the vectors for use according the invention. A "therapeutically effective amount" refers to the amount of the vectors that is effective, at dosages and for periods of time necessary, to achieve the desired therapeutic result, in this case for both prophylaxis and treatment of hearing loss without unacceptable toxicity or undesirable side effects.

A therapeutically effective amount of the vectors can vary according to factors such as the disease state, age, sex, and weight of the subject, and the ability of said compound to elicit a desired response in same. A therapeutically effective amount can also be one in which any toxic or detrimental effects of the claimed compounds are outweighed by the therapeutically beneficial effects. A "prophylactically effective amount" refers to an amount of the vectors of the invention that is effective, at dosages and for periods of time necessary, to achieve the desired prophylactic result. Typically, since a prophylactic dose can be used in subjects prior to or at an earlier stage of disease, the prophylactically effective amount is usually less than the therapeutically effective amount.

Dosage regimens can be adjusted to provide the optimum desired response (e.g., a therapeutic or prophylactic response). For example, a single bolus can be administered, several divided doses can be administered over time or the dose can be proportionally reduced or increased as indicated by the exigencies of the therapeutic situation. Dosage unit form as used herein refers to physically discrete units suited as unitary dosages for the mammalian subjects to be treated; each unit containing a predetermined quantity of the vector compound of the invention calculated to produce the desired therapeutic or prophylactic effect in association with the required pharmaceutical carrier. The specification for the dosage unit forms can be dictated by and directly dependent on (a) the unique characteristics of the vector(s) and the particular therapeutic or prophylactic effect to be achieved, and (b) the limitations inherent in the art of formulating such vector(s) for treating or preventing hearing loss in a subject.

In some embodiments, where first and second AAV particles are to be used, the first and second AAV polynucleotides / particles may be contained within the same composition or within different compositions and may be administered together or separately.

In some embodiments, the composition of the invention contains from 10⁶ to 10¹⁴ particles/mL or from 10¹⁰ to 10¹⁵ particles/mL, or any values there between for either range, such as for example, about 10⁶, 10⁷, 10⁸, 10⁹, 10¹⁰, 10¹¹, 10¹², 10¹³, or 10¹⁴ particles/mL. In one embodiment, the composition of the invention contains more than 10¹³ of AAV particles/mL

In some embodiments, when a first AAV particle comprising a first polynucleotide and a second AAV particle comprising a second polynucleotide are administered, the amount administered is the same for both particles.

Described are treating methods involving the administration of the vector system and pharmaceutical compositions containing same, to patients, especially human patients having a mature auditory system, suffering from DFNB9 deafness. All the embodiments disclosed above apply to said treating methods.

### BRIEF DESCRIPTION OF THE DRAWINGS

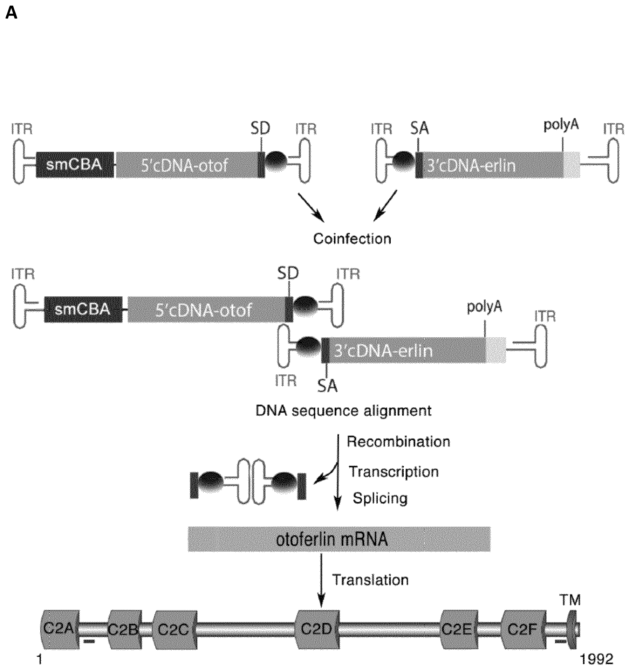

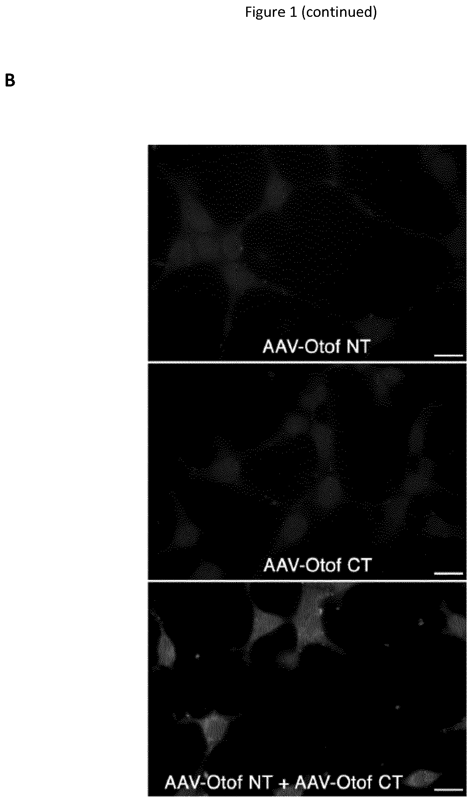

**Figure 1** **shows the expression of otoferlin in HEK293 cells following dual AAV-vector delivery.**
   A) A schematic representation of the recombinant AAV vector pair used in this study, and of the recombination, transcription, splicing, and translation processes producing the full-length protein otoferlin in co-infected cells. The recombinant AAV-Otof NT and AAV-Otof CT vectors contain the 5' and 3' parts of the otoferlin cDNA, respectively. The recombinogenic bridging sequence present in the two recombinant vectors is indicated by a gray sphere. The red bars under the protein diagram denote the two peptides used to produce the antibodies against the N-terminal and C-terminal parts of otoferlin. Abbreviations: ITR, inverted terminal repeats; smCBA, cytomegalovirus immediate early/chicken β-actin chimeric promoter; SA, splice acceptor site; SD, splice donor site; polyA, polyadenylation signal; C2, C2 domain; TM, transmembrane domain. B) HEK293 cells were infected with AAV-Otof NT alone (upper panel), AAV-Otof CT alone (middle panel), or AAV-Otof NT and AAV-Otof CT together (lower panel). They were stained for otoferlin (green) with a polyclonal antibody directed against the C-terminal part of the protein 48 hours later, and cell nuclei were labeled with DAPI (blue). Only co-infected cells produce otoferlin. Scale bars: 15 µm.

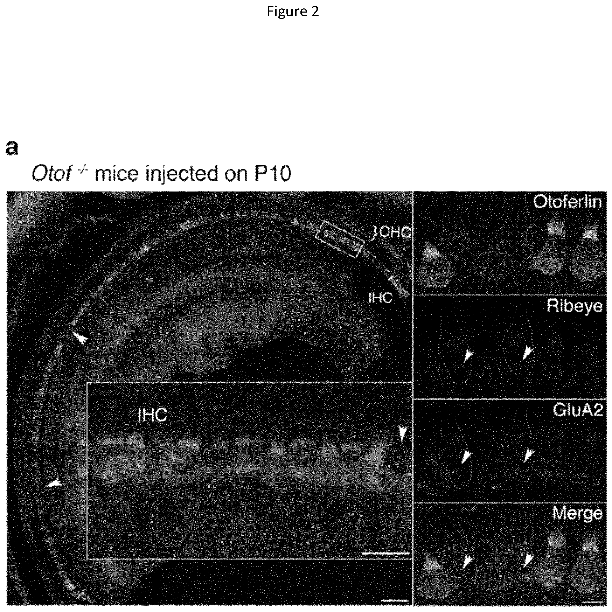

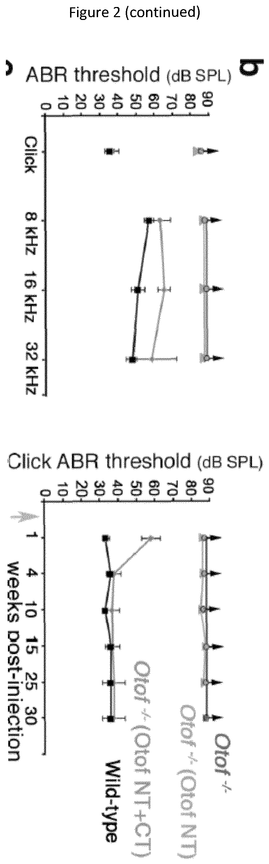

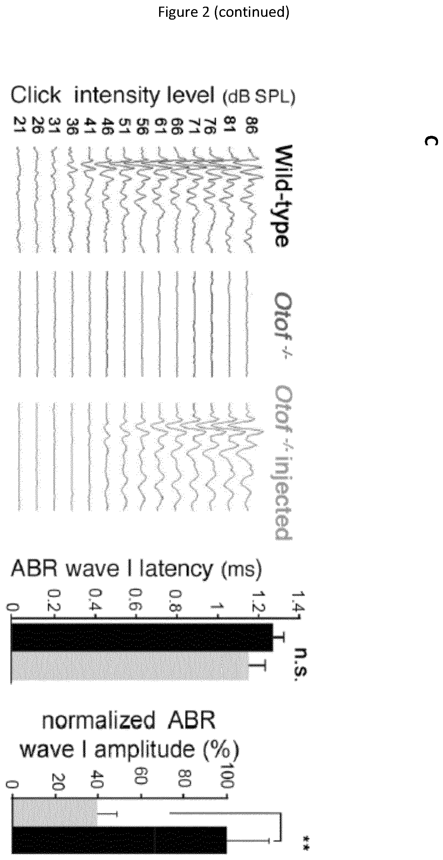

**Figure 2** **shows that the dual AAV-mediated gene therapy in P10 *Otof* -/- mice restores otoferlin expression and prevents deafness.**
   (a) Left panel: Mosaic confocal image of the middle and apical turns of the injected cochlea immunostained for otoferlin on P70 (green). Cell nuclei are stained with DAPI (blue). A large proportion of the IHCs, but none of the outer hair cells (OHC), express otoferlin. Arrowheads indicate nontransduced IHCs. Inset: Higher magnification of the boxed area. Scale bars: 50 µm and 10 µm (inset). Right panel: Images of IHCs co-immunostained for otoferlin (green), the ribbon protein ribeye (blue), and the GluA2 subunit of post-synaptic glutamate receptors (red). Synaptic active zones have a normal distribution in transduced IHCs expressing otoferlin, whereas they tend to form clusters (arrowheads) in non-transduced IHCs (indicated by dashed lines). Scale bar: 5 µm. (**b)** Left panel: Four weeks after the dual AAV injection, *Otof* -/- mice displayed ABR thresholds in response to clicks or tone-bursts at frequencies of 8 kHz, 16 kHz, and 32 kHz (green dots, *n* = 8) close to those of wild-type mice (black dots, *n* = 8). By contrast, *Otof -*/*-* mice receiving AAV-Otof NT (orange dots, *n* = 3) or no injection (blue dots, *n* = 6) had no identifiable ABR waves up to sound intensity levels of 86 dB SPL. Right panel: In the *Otof -*/*-* mice treated on P10 (arrow), the hearing thresholds for click stimuli were stable for at least six months after recovery, **(c)** Left panel: ABR traces, recorded three weeks after therapeutic injection, in a wild-type mouse, an *Otof* -/- mouse (*Otof* -/-), and a rescued *Otof* -/- mouse *(Otof* -/- injected), showing similar waveforms in the wild-type and rescued mice. Right panel: bar graph showing the latency and normalized amplitude of ABR wave I in rescued *Otof -*/*-* mice (grey, *n* = 8) and wild-type mice (black, *n* = 5).

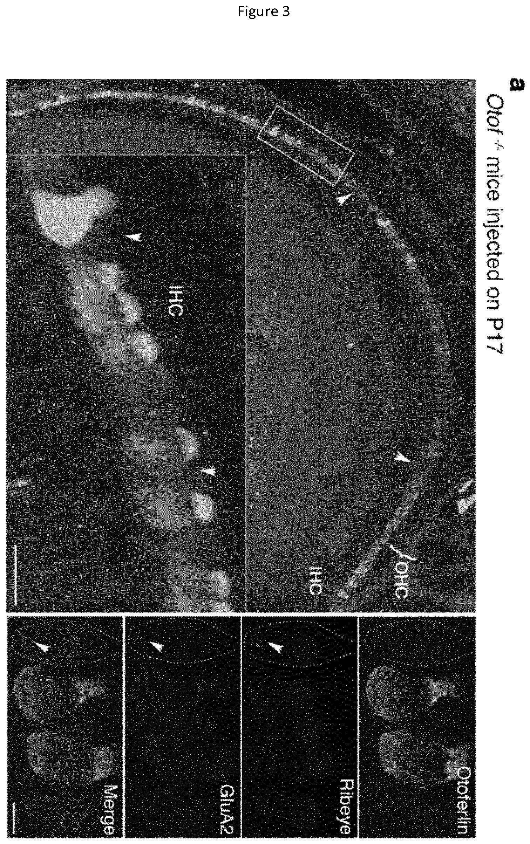

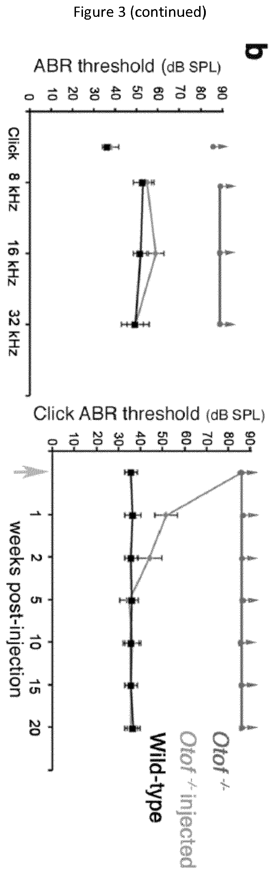

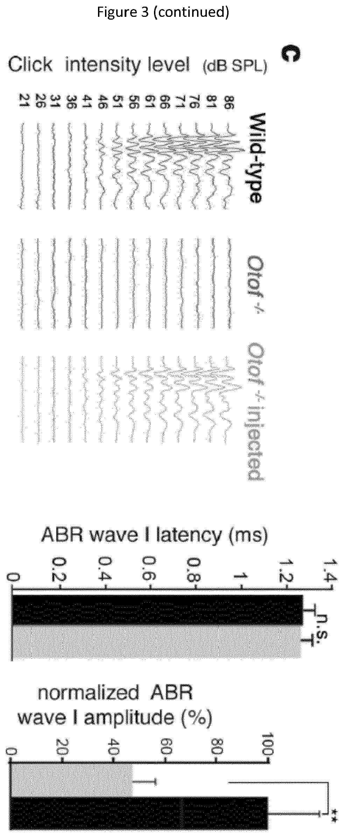

**Figure 3** **shows that dual AAV-mediated gene therapy in *Otof* -/- mice on P17 durably restores otoferlin expression and hearing.**
   **(a)** Left panel: Mosaic confocal image of the middle and apical turns of the injected cochlea, immunostained for otoferlin (green) on P80. Cell nuclei are stained with DAPI (blue). Most IHCs express otoferlin, whereas outer hair cells (OHC) do not. Arrowheads indicate non-transduced IHCs. Inset: Higher magnification of the boxed area. Scale bars: 50 µm and 10 µm (inset). Right panel: Images of IHCs coimmunostained for otoferlin (green), the ribbon protein ribeye (blue), and the GluA2 subunit of postsynaptic glutamate receptors (red). Synaptic active zones have a normal distribution in transduced IHCs expressing otoferlin, whereas they tend to form clusters (arrowheads) in non-transduced IHCs (indicated by dashed lines). Scale bar: 5 µm. (**b)** Left panel: ABR thresholds of untreated *Otof* -/- mice (blue, *n* = 5), treated *Otof* -/- mice (green, *n* = 5), and wild-type mice (black, *n* = 5) in response to clicks or tone-burst stimuli at frequencies of 8, 16, and 32 kHz, four weeks after intracochlear injection of the recombinant vector pair in the treated mice. Right panel: time course of hearing recovery in *Otof -*/*-* mice receiving injections on P17 (arrow). Hearing restoration to near-wild type levels is maintained for at least twenty weeks post-injection. **(c)** Left panel: ABR traces, recorded two weeks after therapeutic injection, in a wild-type mouse (black), an *Otof* -/- mouse (*Otof* -/-), and a rescued *Otof* -/- mouse *(Otof* -/- injected), showing similar waveforms in the wild-type and rescued mice. Right panel: bar graph showing that the latency of ABR wave I in rescued *Otof* -/- mice *(n* = 5) is similar to that in wild-type mice *(n* = 5), whereas its normalized amplitude is about half that in wild-type mice.

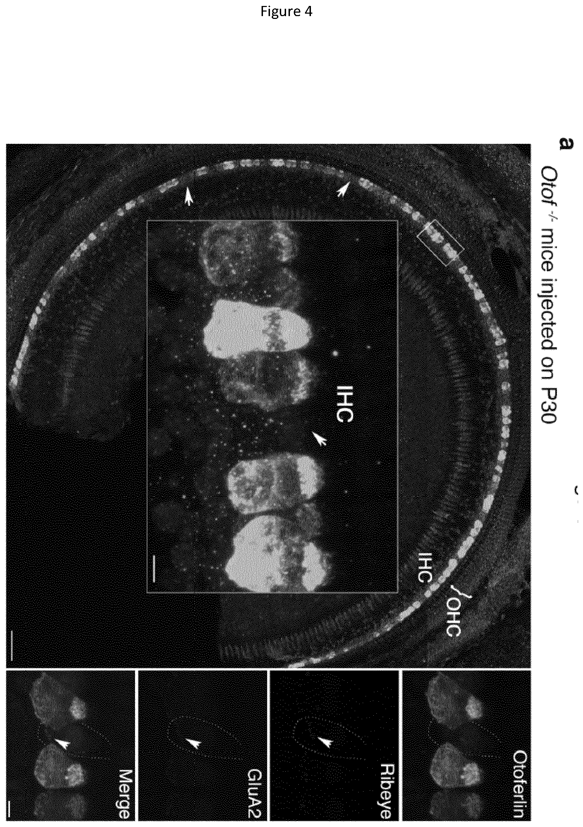

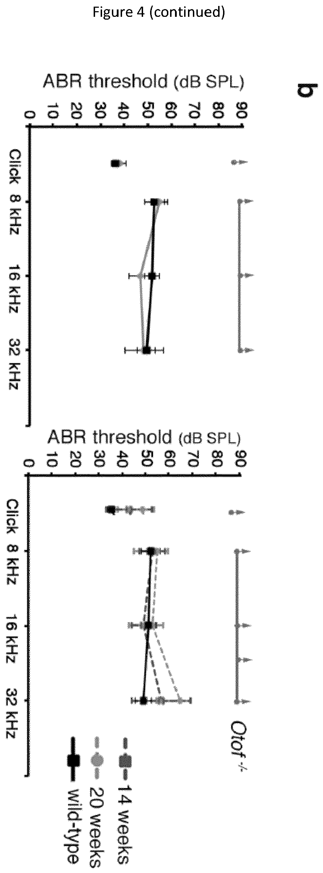

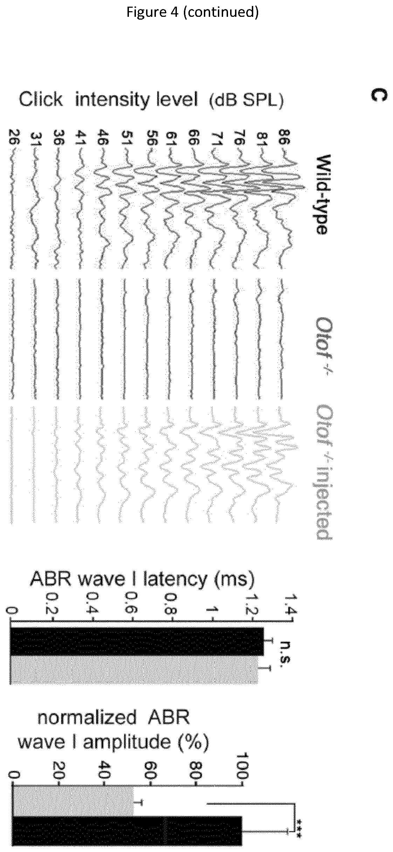

**Figure 4** **shows that dual AAV-mediated gene therapy in *Otof* -/- mice on P30 restores otoferlin expression and hearing in** a **sustained manner.**
   **(a)** Left panel: Mosaic confocal image of the middle and apical turns of the injected cochlea, immunostained for otoferlin on P40 (green). Cell nuclei are stained with DAPI (blue). Most IHCs express otoferlin, whereas outer hair cells (OHC) do not. Arrowheads indicate non-transduced IHCs. Inset: Higher magnification of the boxed area. Scale bars: 50 µm and 10 µm (inset). Right panel: Images of IHCs coimmunostained for otoferlin (green), the ribbon protein ribeye (blue), and the GluA2 subunit of postsynaptic glutamate receptors (red). Synaptic active zones have a normal distribution in transduced IHCs expressing otoferlin, whereas they tend to form clusters (arrowheads) in non-transduced IHCs (indicated by dashed lines). Scale bar: 5 µm. (**b)** ABR thresholds of untreated *Otof* -/- mice (blue, *n* = 3), treated *Otof-*/*-* mice (green, *n* = 3) and wild-type mice (black, *n* = 3) in response to clicks or tone-burst stimuli at frequencies of 8, 16, and 32 kHz, three weeks (left panel), fourteen weeks, and twenty weeks (right panel) after intracochlear injection of the recombinant vector pair in the treated mice. In these mice hearing restoration to near-wild type levels is maintained for at least twenty weeks post-injection. **(c)** Left panel: ABR traces, recorded seven weeks after therapeutic injection, in a wild-type mouse (black), an *Otof* - /- mouse *(Otof* -/-), and a rescued *Otof -*/*-* mouse *(Otof* -/- injected), showing similar waveforms in the wild-type and rescued mice. Right panel: bar graph showing that the latency of ABR wave I in rescued *Otof* -/- mice *(n* = 3) is similar to that in wild-type mice *(n* = 3), whereas its normalized amplitude is about half that in wild-type mice.

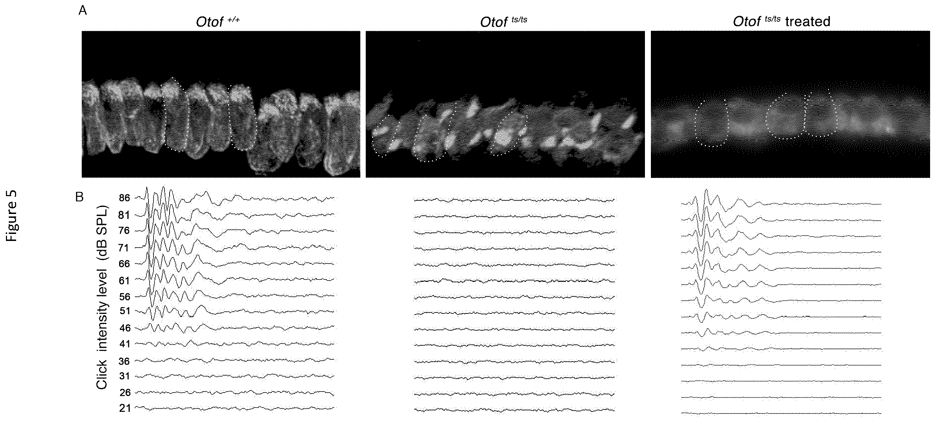

**Figure 5** shows the dual AAV-mediated gene therapy in *Otof*^{*ts*/*ts*} mice restores otoferlin normal expression and hearing. A) Confocal image of IHCs (outlined by dashed lines) located in the mid-turn cochlea from a wild-type (*Otof*^{+/+}) mouse (left panel), an *Otof*^{*ts*/*ts*} mouse (middle panel), and a treated *Otof*^{*ts*/*ts*} (right panel) mouse, immunostained for otoferlin (green). While otoferlin shows abnormal aggregation at the IHC base in the non-treated *Otof* ^{*ts*/*ts*} mouse, its expression in the IHCs of the treated mice is nearly normal. B) ABR waveforms, recorded four weeks after therapeutic injection, in a wild-type mouse (black), an *Otof*^{*ts*/*ts*} mouse (blue), and a rescued *Otof* ^{*ts*/*ts*} mouse (green), showing similar waveforms in the wild-type and rescued mice, while no ABR waves are detected in the untreated mutant.
The schema on **Figure 6** discloses the differential maturation of hearing system in humans and in mice (Shnerson and Willott, J. Comp. Physiol. Psychol. 1980 Feb; 94(1):36-40).
**Figure 7** describes some of the mutations of the DFNB9 gene that have been identified so far. These mutations underlay recessive form of the prelingual deafness DFNB9.
**Figure 8** shows (**A**) the protein aggregation and misfolding of Otoferlin in the inner hair cells of *Otof*^{*ts*/*ts*} mouse and (**B**) the auditory brainstem responses (ABRs) in *Otof* ^{+/}*^{ts},* and *Otof*^{*ts*/*ts*} mice (see also Figure 5).
**Figure 9** discloses the effect of unilateral injection of the AAV-Otof NT plus AAV-Otof CT recombinant vector pair on *Otof*^{*ts*/*ts*} mice. 5 weeks after the injection, the sensory epithelium of the treated cochleas of three *Otof*^{*ts*/*ts*} mice was microdissected and immunolabeled for otoferlin. Otoferlin expression in the IHC of the treated cochlea has been measured and compared with its expression in *Otof*^{*ts*/*ts*} non-treated mice (see also Figure 5).
**Figure 10** discloses the voltage-activation curve of *I*_{Ca} (***A***) and corresponding Δ*C*ₘ responses (***B***) in wild-type, Otof^{ts/ts} and Otof^{ts/ts} treated IHC. Changes in cell membrane capacitance (Δ*C*ₘ) were used to monitor fusion of synaptic vesicles during exocytosis.

### EXAMPLES

### I. Material and Methods

### Animals

Otoferlin knockout *(Otof* -/-) mice produced in the C57BL/6 strain (Roux I. et al, Cell, 127, 277-289 (2006) were backcrossed with FVB mice for more than ten generations to obtain a homogeneous FVB genetic background, as this background, unlike the C57BL/6 background, is associated with stable hearing thresholds in the first ten months of life (Kommareddi, P., et al. J Assoc Res Otolaryngol 16, 695-712 (2015)). Recombinant AAV2 vectors were delivered to the *Otof* -/- mice in an FVB genetic background. All procedures and animal handling complied with *Institut National de la Santé et de la Recherche Médicale, Institut Pasteur,* and NIH welfare guidelines, and approved protocol requirements at the University of California, San Francisco. Before surgery, mice were anesthetized by intraperitoneal injection of a mixture of ketamine hydrochloride (Ketaset, 100 mg/kg), xylazine hydrochloride (Xyla-ject, 10 mg/kg), and acepromazine (2 mg/kg). The depth of anesthesia was checked by monitoring the deep tissue response to toe pinch. Before and every 24 hours after surgery for a week, the mice received subcutaneous injection of carprofen (2 mg/kg) to reduce inflammation and pain. Animals were monitored for signs of distress and abnormal weight loss after surgery.

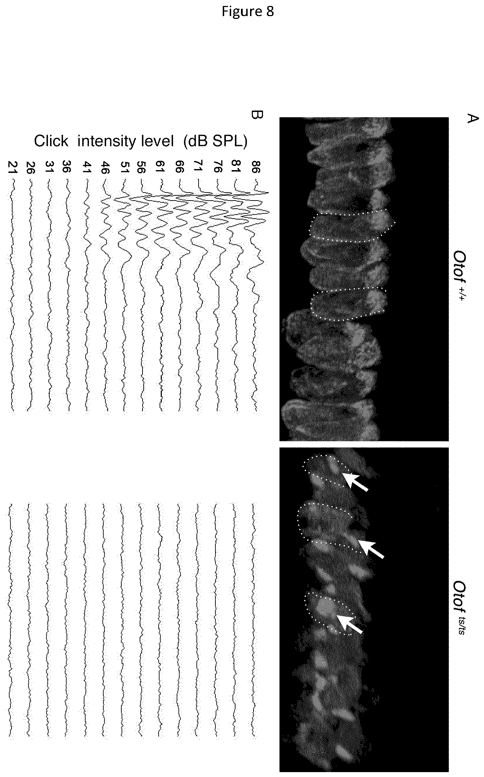

A mouse model carrying a thermosensitive mutation in the C2F domain (*Otof*^{*ts*/*ts*}) (p.E1804del) was generated. The *Otof*^{*ts*/*ts*} mice are profoundly deaf. The results shown on Figure 8 highlight that the distribution of otoferlin in the IHCs of these mice is abnormal/strongly perturbed: the protein is aggregated and misfolded in the inner hair cells. Moreover, when auditory brainstem responses (ABRs) are recorded in *Otof* ^{*+*/}*^{ts},* and *Otof*^{*ts*/*ts*} mice to monitor the electrical response of the primary auditory neurons and the successive neuronal relays of the central auditory pathway to a click stimulus, it is observed that, at the age of one month, ABR characteristic waveform is obtained for *Otof*^{*+*/*ts*} mice at the various intensities tested (40-86 dB), but no response is elicited in *Otof*^{*ts*/*ts*} mice, even at 100 dB.

### Recombinant AAV2 vector constructs and packaging

The full-length coding sequence of the murine otoferlin cDNA (*Otof1* isoform 1;NM_001100395.1) was divided into a 5' fragment (nucleotides 1-2448) and a 3' fragment (nucleotides 2449-5979), and these fragments were synthesized (Genscript, Piscataway, NJ). The 5' construct contained the 5' part of the *Otof1* cDNA (encoding amino acids 1-816, which includes the C2A, C2B, and C2C domains of the protein) and a splice donor (SD) site, and the 3' construct contained the 3' part of the *Otof1* cDNA (encoding amino acids 817- 1992, which includes the C2D, C2E, C2F and transmembrane domains of the protein), a splice acceptor site (SA). In addition, both constructs contain the alkaline phosphatase recombinogenic bridging sequence [Lay Y et al, Hum Gene Ther 17, 1036-1042 (2006); Ghosh A. et al, Hum Gene Ther 22, 77-83 (2011); Dyka F.M. et al, Hum Gene Ther Methods 25, 166-177 (2014)]. Recognition sites for *Not*I/*Nhe*I and *Mf*eI/*Kpn*I restriction endonucleases were added to these constructs, which were then inserted into an AAV pTR22 vector plasmid as previously described [Lay Y et al, Hum Gene Ther 17, 1036-1042 (2006); Ghosh A. et al, Hum Gene Ther 22, 77-83 (2011); Dyka F.M. et al, Hum Gene Ther Methods 25, 166-177 (2014)], producing a pair of recombinant vectors referred to as AAV-Otof NT and AAV-Otof CT. An additional recombinant vector containing the green fluorescent protein (GFP) cDNA was engineered to serve as a positive control of cell transduction. The recombinant vectors were packaged in the AAV2 quadY-F capsid (Petrs-Silva H. et al, Molecular therapy: the journal of the American Society of Gene Therapy 19, 293-301 (2011), and recombinant viruses were purified and titered by the University of Florida Ocular Gene Therapy Core, as previously described [Zolotukhin S. et al, Methods 28, 158-167 (2002); Jacobson SG et al., Molecular therapy : the journal of the American Society of Gene Therapy 13, 1074-1084 (2006)].

### Transgene expression in transfected HEK293 cells

HEK293 cells were grown in 6-well plates on polylysine-coated coverslips in Dulbecco's modified Eagle's medium supplemented with 1x non-essential amino acids and 10% fetal bovine serum (Gibco), and penicillin-streptomycin (Pen/Strep, Invitrogen). On the next day, cells were infected as previously described (Lopes V.S. et al, Gene Ther 20, 824-833 (2013). Briefly, the coverslips with the cells at 75% confluence were incubated in 200 µl of serum-free medium containing either one or both AAV2-Otof recombinant viruses (10 000 genome-containing particles/cell for each vector) at 37 °C with 5% CO2. Two hours later, 1 ml of complete medium was added. The next day the medium was changed, and cells were incubated for an additional 48 hours. The cells were fixed with 4% paraformaldehyde in 0.1 M phosphate buffered saline (PBS), pH 7.4, at 4°C for two hours, rinsed three times with PBS, and incubated with 0.25% Triton X-100 and 5% normal goat serum in PBS at room temperature for one hour. The cells were then incubated with previously characterized rabbit polyclonal antibodies, 14cc and C19, directed against the N-terminal part (amino acids 196-211) and the C-terminal part (amino acids 1848-1978) of otoferlin, respectively (Roux I. et al, Cell, 127, 277-289 (2006) (dilution 1:200) at 4°C overnight. The samples were rinsed twice with PBS, and incubated with Cy3-conjugated goat anti-rabbit IgG secondary antibody (Life Technologies, dilution 1:2000) in PBS at room temperature for two hours. The samples were then rinsed twice in PBS, stained with 4',6-diamidino-2-phenylindole (DAPI) to visualize cell nuclei, mounted on a glass slide with a drop of Fluorsave medium (Biochem Laboratories, France), and observed with an Olympus confocal immunofluorescence microscope.

### Vector delivery to the cochlea

The virus was delivered to the left cochlea as previously described (Akil O. et al, Neuron 75, 283-293 (2012)). Anesthetized *Otof -*/*-* mice received an injection of the AAV2-Otof vector pair through the round window membrane into the scala tympani of the cochlea on P10, P17, or P30. The ear was approached via a dorsal incision (Duan M et al, Gene Ther 11 Suppl 1, S51-56 (2004). A small hole was made in the bulla with an 18G needle, and expanded with forceps. The round window membrane was gently punctured with a borosilicate capillary glass pipette, which was then removed. When perilymph efflux stopped, a fixed volume (2 µl) containing the AAV2-Otof NT (6.3 × 10¹² vg/ml) and AAV2-Otof CT (4.5 × 10¹² vg/ml) vector pair was injected into the scala tympani with a fine glass micropipette (outer tip diameter of 10 µm) over a period of one minute. The pipette was pulled out, and the niche was rapidly sealed with fascia and adipose tissue. The wound was sutured in layers with a 6-0 absorbable chromic suture (Ethicon).

### Auditory testing

Auditory testing was carried out in anesthetized *Otof*^{+/+} mice, *Otof*^{-/-} mice, and rescued *Otof*^{-/-} mice at different time points, in a sound-proof chamber, as previously described (Akil O. et al, Neuron 75, 283-293 (2012). The mice were anesthetized with an intraperitoneal injection of a mixture of ketamine hydrochloride (Ketaset, 100 mg/ml) and xylazine hydrochloride (Xyla-ject, 10 mg/ml), with subsequent injections of one fifth of the initial dose if required. Body temperature was maintained with a heating pad and monitored with a rectal probe throughout recording. Auditory brainstem responses (ABR) were recorded with the TDT BioSig III system (Tucker Davis Technologies) and three subdermal needle electrodes located on the mouse scalp: one at the vertex, one below the pinna of the left ear (reference electrode), and one below the contralateral ear (ground electrode). The sound stimuli were clicks (5 ms duration, 31 Hz) and tone pips at 8, 16, and 32 kHz (10 ms duration, cosine squared shaping, 21 Hz). They were delivered in free-field conditions, with monaural response recording from the left ear (the right ear was blocked during the recording). For each sound stimulus, electroencephalographic (EEG) activity was recorded for 20 ms at a sampling rate of 25 kHz, with filtering (0.3-3 kHz). EEG waveforms for 512 stimuli and 1000 stimuli were averaged for clicks and tone bursts, respectively. The sound stimulus intensity was decremented in 5 dB sound pressure level (SPL) intervals down from the maximum intensity tested (86 dB SPL). The hearing threshold was defined as the lowest stimulus level at which ABR peaks for waves I-V were clearly and repeatedly present upon visual inspection. These threshold evaluations were confirmed by the offline analysis of stored waveforms. The latency of ABR wave I was measured as the time interval between the click stimulus and the peak amplitude of wave I. In addition, the values of wave I peak amplitudes on the ABR traces were normalized against the mean value in control wild-type mice (taken as 100 %) for a comparison between rescued *Otof -*/*-* mice and wild-type mice.

### Inner hair cell and synaptic ribbon counts

Mouse cochleas were perfused with 4% paraformaldehyde in 0.1 M PBS (pH 7.4) and incubated in the same fixative at 4°C for two hours. The cochleas were rinsed three times with PBS, and decalcified by incubation with 5% ethylenediamine tetra-acetic acid (EDTA) in 0.1 M PBS at 4°C overnight. The cochlear sensory epithelium (organ of Corti) was microdissected into a surface preparation, preincubated in 0.25% Triton X-100 and 5% normal goat serum in PBS (blocking buffer) at room temperature for one hour, and incubated with the primary antibody at 4°C overnight. The following antibodies were used: rabbit antiotoferlin C-terminal part (C19, 1:250 dilution) 1, mouse (lgG1) anti-CtBP2/ribeye, mouse (IgG2a) anti-glutamate receptor subunit A2 (Millipore, 1:200 dilution), and rabbit anti-GFP (Invitrogen, A11122; 1:250 dilution). The samples were rinsed three times in PBS, and incubated with the appropriate secondary antibody: Alexa Fluor 488-conjugated anti-mouse IgG1, Alexa Fluor 568-conjugated anti-mouse IgG2a (Life Technologies, 1:1000 dilution), or Atto Fluor 647-conjugated anti-rabbit IgG (Sigma, 1:200 dilution). The samples were washed three times in PBS, and mounted on a glass slide in one drop of Fluorsave, with DAPI to stain cell nuclei. Fluorescence confocal z-stacks of the organ of Corti were obtained with an LSM 700 confocal microscope (Zeiss, Oberkochen, Germany) equipped with a high-resolution objective (numerical aperture of 1.4, 60 × oil-immersion objective). Images were acquired in a 512 × 512 or 1024 × 1024 raster (pixel size = 0.036 µm in *x* and *y*) with 0.2 µm z steps. Inner hair cells (IHCs) producing otoferlin and synaptic ribbons were counted by the 3D rendering of z-stacks of up to 20 confocal images. To calculate the proportion of IHCs expressing the otoferlin transgene, we divided the total number of IHCs producing otoferlin by the total number of IHCs identified by their DAPI-stained cell nuclei (a minimum of 150 consecutive analyzed).

### Reverse transcriptase-polymerase chain reaction (RT-PCR)

Total mRNA was extracted (Trizol, Invitrogen) from the left cochleas of six *Otof* +/+ mice and six *Otof* -/- mice rescued on P10. Reverse transcription (RT) was carried out with oligodT primers and superscript II RNase H⁻ (Invitrogen) at 42°C for 50 minutes. Two microliters of the RT reaction product were used for the polymerase chain reaction (PCR; *Taq* DNA polymerase, Invitrogen) consisting of 35 cycles (94°C for 30 s, 60°C for 45 s, 72°C for 60 s) with final extension at 72°C for 10 minutes. The PCR primer pair (forward primer TGTCTCAGAGCTCCGAGGCA (SEQ ID NO:14) and reverse primer ATCGTGGAGGAGGAACTGGGCA (SEQ ID NO:15) was designed to amplify a 2676 bp intermediate fragment (nucleotides 27 to 2702) of the otoferlin cDNA (GenBank accession number NM_001100395.1) encompassing the junction between the AAV-Otof NT and AAV-Otof CT inserts. PCR products were purified by electrophoresis on a 2% agarose gel containing 0.5 mg/ml ethidium bromide (Qiaquick gel extraction kit, QIAGEN), sequenced (Elim Biopharmaceuticals), and checked for sequence identity to the otoferlin cDNA sequence.

### Statistical analyses

Data are expressed as the mean ± standard deviation (SD). All statistical analyses were carried out with the non-parametric Mann-Whitney *U* test. Statistical significance is indicated in the figures as follows: n.s., not significant; *, *p* < 0.05; **, *p* < 0.01; ***, *p* < 0.001.

### II. Results

An AAV2-based vector was engineered to express the green fluorescent protein (GFP) gene under the control of a chimeric CMV-chicken β-actin promoter. This expression cassette was packaged in the AAV2 quadY-F capsid wherein four surface tyrosine (Y) residues of the AAV2 capsid have been replaced by phenylalanine (F) residues, which was shown to increase the efficiency of gene transfer in the retina (Petrs-Silva H. et al, Molecular therapy: the journal of the American Society of Gene Therapy 19(2):293-301 (2011)). The recombinant virus was injected through the round window membrane into the left cochlea of five wild-type mice on P2. GFP immunostaining of the sensory epithelium three weeks after injection revealed the transduction of various types of cells including IHCs. The transduction rate for IHCs was 78 ± 6% (mean ± SD), demonstrating the suitability of this AAV serotype to deliver therapeutic genes to these cells (not shown). The coding sequence of the murine otoferlin cDNA was split into a 5' fragment (Otof NT, nucleotides 1-2448) and a 3' fragment (Otof CT, nucleotides 2449-5979), each of which was inserted into an AAV vector carrying a recombinogenic bridging sequence (Ghosh A. et al, Hum Gene Ther 22(1):77-83 (2011); Dyka FM et al, Hum Gene Ther Methods 25(2):166-177 (2014)). The AAV-Otof NT recombinant vector carries the 5' part of the cDNA followed by a splice donor site, and the AAV-Otof CT recombinant vector carries a splice acceptor site followed by the 3' part of the cDNA (see Methods and Figure 1). Each of these recombinant vectors was packaged in the AAV2 quadY-F capsid. HEK293 cells were infected with AAV-Otof NT, AAV Otof CT, or both recombinant viruses, and immunostained for otoferlin 48 hours later. Two different antibodies were used, directed against the C-terminal part or the N-terminal part of the protein (Roux I, et al, Cell 127:277-289 (2006)), and obtained identical results. Otoferlin was detected only in cells infected simultaneously with both viruses, thus indicating that the two vectors were able to recombine and generate concatemers via their inverted terminal repeats, with correct splicing of the resulting transcript to produce the protein (Figure 1).

A single unilateral injection of the AAV-Otof NT plus AAV-Otof CT recombinant vector pair was administered to *Otof* -/- mice through the round window membrane into the left cochlea, before (on P10) or after hearing onset. Injections after hearing onset were carried out at one of two different time points, P17 and P30, because the maturation of IHC ribbon synapses is still underway at P17 (Kros CJ et al, Nature 394(6690):281-284 (1998); Wong AB et al, EMBO J 33(3):247-264 (2014)), whereas the cochlea is mature at P30 (Song L. et al, J Acoust Soc Am 119(4):2242-2257 (2006)). Eight weeks after the injection of the recombinant vector pair on P10, the sensory epithelium of the treated cochleas of three *Otof* -/- mice was microdissected and immunolabeled for otoferlin (with an antibody directed against the C-terminal part of the protein) to estimate the IHC transduction rate. The protein was detected in more than 60% of the IHCs (64 ± 6%, mean ± SD, *n* = 3 cochleas), but not in other cell types (Figure 2a, left panel). This result provides evidence that a large cDNA can effectively be reconstituted in cochlear sensory cells upon the local delivery of a recombinant AAV-vector pair *in vivo,* with sustained, widespread production of the protein by a large proportion of the cells. The accuracy of the pre-mRNA splicing process in transduced cells was checked by RT-PCR and sequence analysis of a large fragment of the otoferlin transcript encompassing the junction between the Otof NT and Otof CT cDNAs (not shown) was made.

Auditory brainstem response (ABR) recordings in the mice four weeks after the P10 injection demonstrated a substantial restoration of hearing thresholds in response to click and tone-burst stimuli (8, 16, and 32 kHz) in all the treated mice (*n* = 8), but no restoration in the *Otof* -/- mice receiving either AAV-Otof NT or AAV-Otof CT alone (*n* = 3 each), or in the absence of injection (*n* = 6) (Figures 2b,2c). The ABR thresholds for both click and tone-burst stimuli in the treated mice were similar to those of control wild-type mice (*n* = 8; Mann-Whitney U test, *p* >0.15 for all comparisons). The long-term efficacy of gene therapy was evaluated by carrying out ABR recordings in response to clicks at several post-injection time points between 1 and 30 weeks. From the fourth week onward, the ABR thresholds of the treated mice did not differ significantly from those of wild-type mice (Mann-Whitney U test, *p* > 0.05 for comparisons at all stages) (Figure 2b). However, the amplitudes of ABR wave I, which reflects the electrical responses of primary auditory neurons to the sound stimulus, were 39 ± 7% (mean ± SD) of the mean value for wild-type mice (Mann-Whitney U test, *p* = 0.002), whereas wave I latencies (1.15 ± 0.09 ms) were similar to those in wild-type mice (1.27 ± 0.05 ms; Mann-Whitney U test, *p* = 0.06) (Figure 2c).

Thirty weeks after the injection, six of the eight mice receiving injections on P10 still had hearing thresholds within 10 dB of those of wild-type mice. Gene therapy before hearing onset therefore prevents deafness in *Otof* -/- mice. The Inventors have previously shown that about half of the IHC ribbons degenerate in *Otof -*/*-* mice (Roux I, et al, Cell 127:277-289 (2006)). The numbers of presynaptic ribbons (together with postsynaptic glutamate receptors) was analysed in the transduced IHCs and the nontransduced IHCs of treated *Otof* -/- cochleas eight weeks after the injection on P10, by immunofluorescence and 3D confocal microscopy imaging (Figure 2a, right panel). The number of ribbons per IHC in transduced cells (12.5 ± 1.8, mean ± SD, *n* = 48 cells from 3 mice) was almost twice higher than in non-transduced cells (6.9 ± 1.3, *n* = 48 cells from 3 mice; Mann-Whitney U test, *p* < 10⁻⁴), but remained lower than in wild-type IHCs (16 ± 1.3, *n* = 48 cells from 3 mice; Mann-Whitney U test, *p* < 10⁻⁴), potentially accounting for the incomplete recovery of wave I amplitude on ABR recordings.

After injection of the recombinant vector pair into the cochlea of P17 or P30 *Otof -*/*-* mice, otoferlin was detected in IHCs throughout the treated cochlea, but not in IHCs of the contralateral cochlea (not shown). IHC transduction rates were similar in the two groups of mice (82 ± 9% and 85 ± 7%, for *n* = 5 and *n* = 3 cochleas treated on P17 and P30, respectively), and higher than those in mice receiving injections on P10 (Mann-Whitney U test, *p* < 0.05 for both comparisons) (Figures 3a and 4a). ABR recordings four weeks after injection showed hearing recovery in all the mice receiving injections on P17 (*n* = 5), with ABR thresholds in response to clicks or tone-burst stimuli remarkably similar to those in wild-type mice (*n* = 5; Mann-Whitney U test, *p* > 0.2 for all comparisons). Hearing thresholds in response to clicks remained unchanged for 20 weeks after injection, demonstrating a sustained restoration of hearing in these mice despite a mean ABR wave I amplitude about half that in wild-type mice (47 ± 10%) (Figures 3b,c).

Likewise, *Otof-*/*-* mice receiving injections on P30 displayed a similar recovery of hearing as early as three weeks after the injection, with ABR thresholds in response to clicks or tone-burst stimuli persisting at the wild-type level for 20 weeks post-injection (*n* = 3, Mann-Whitney U test, *p* > 0.5 for comparisons at all stages), despite a mean ABR wave I amplitude about half (55 ± 10%) that in wild-type mice (Fig. 4b,c). The numbers of presynaptic ribbons (together with postsynaptic glutamate receptors) was analysed in the transduced IHCs and the non-transduced IHCs of *Otof* -/- cochleas treated on P17 and on P30, by immunofluorescence and 3D confocal microscopy imaging (Figures 3a and 4a). The numbers of ribbons per IHC in transduced cells (10.0 ± 1.3, mean ± SD, *n* = 48 cells from 3 mice treated on P17 and analysed on P80, and 8.9 ± 2.3, *n* = 48 cells from 3 mice treated on P30 and analyzed on P40) were higher than in non-transduced cells from the same cochleas (6.2 ± 1.3, *n* = 48 cells, and 5.8 ± 0.7, *n* = 48 cells, respectively; Mann-Whitney U test, *p* < 10-4 for both comparisons), but they were lower than in IHCs of 10-week old wild-type mice (16 ± 1.3, *n* = 48 cells from 3 mice; Mann-Whitney U test, *p* < 10-4 for both comparisons). As the number of ribbons per IHC was already markedly reduced in untreated *Otof* -/- mice analyzed on P15 (8.2 ± 1.0, *n* = 48 cells from 3 mice), and remained unexpectedly stable in the non-transduced IHCs of treated mice at later stages (see above the values for P40, P70, and P80), it can be inferred that gene therapy in the IHCs of *Otof -*/*-* mice increased the number of ribbons by promoting their production rather than preventing their degeneration.

Using the dual AAV gene therapy disclosed above, administered at p30 in the animals, it has also been possible to restore both the normal distribution of otoferlin and the hearing function to near normal ABR thresholds in a mouse model carrying a human thermosensible mutation in its DFNB9 gene (*Otof*^{*ts*/*ts*} mice, Figure 5).

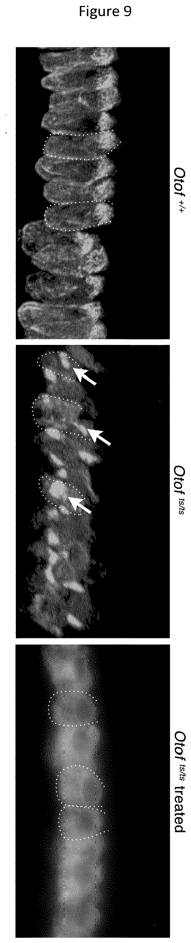

More precisely, it has been shown that dual viral gene therapy overwrites otoferlin aggregates due to the human thermosensitive mutation in said DFNB9 mouse model (Figure 9). In this experiment, a single unilateral injection of the AAV-Otof NT plus AAV-Otof CT recombinant vector pair was administered to *Otof*^{*ts*/*ts*} mice through the round window membrane into the left cochlea, after hearing onset were carried out at P30. 5 weeks after the injection, the sensory epithelium of the treated cochleas of three *Otof*^{*ts*/*ts*} mice was microdissected and immunolabeled for otoferlin (see Figure 9). Otoferlin expression in the IHC (dashed lines) of the treated cochlea was found nearly normal when compared to the otoferlin aggregates in non-treated cochlea *of the Otof* ^{*ts*/*ts*} mouse (arrows).

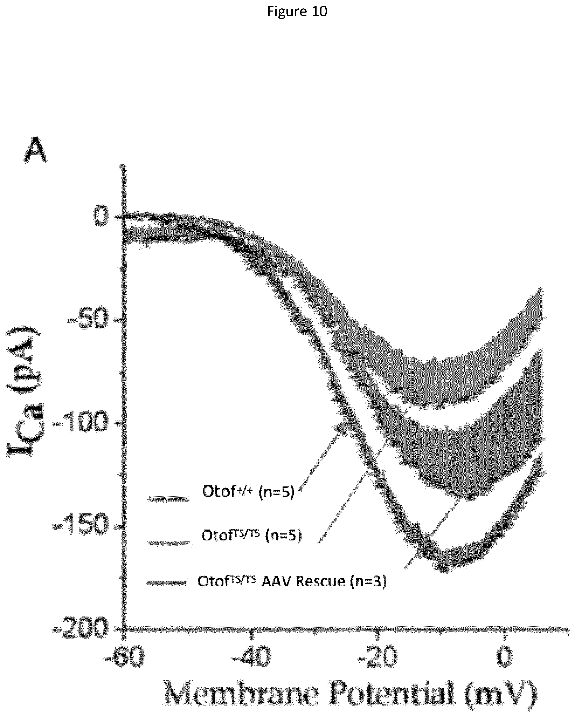

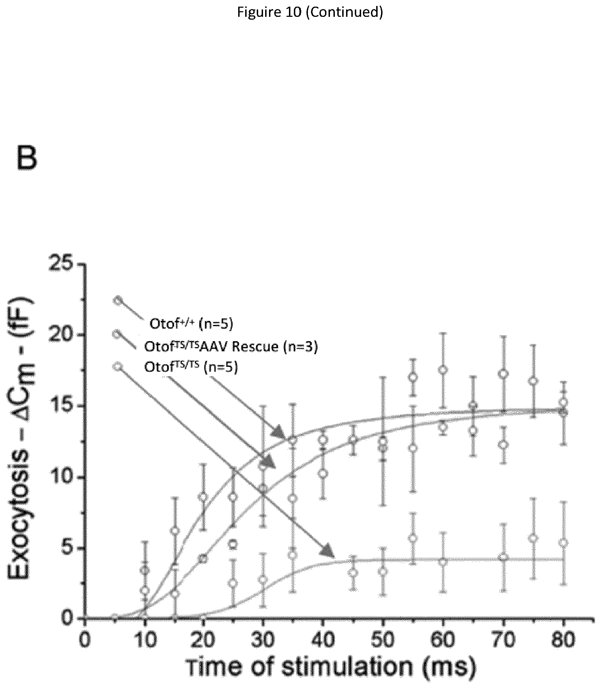

It has been eventually found that said dual viral gene therapy restores the calcium currents and exocytosis of the IHC in this animal model, as shown on Figure 10.

## Claims

1. A vector system comprising at least two AAV particles, each of them comprising a polynucleotide comprising a partial coding sequence that encodes i) the N-terminal part of the Otoferlin polypeptide or of a functional fragment thereof, for one, and ii) the C-terminal part of the Otoferlin polypeptide or of a functional fragment thereof, for the other, said vector system allowing the expression of the full-length Otoferlin polypeptide, or of a functional fragment thereof, in inner hair cells, for use for treating patients suffering from DFNB9 deafness or preventing DFNB9 deafness in patients having DFNB9 mutations, wherein said patients are human having a developed and mature auditory system.

2. The vector system for use according to claim 1, wherein said Otoferlin polypeptide has the sequence SEQ ID NO:1.

3. The vector system for use according to claim 1, wherein said Otoferlin polypeptide has the sequence SEQ ID NO:5.

4. The vector system for use according to any of claims 1 to 3, wherein said human patients are new born babies, toddlers, infants, teenagers or adults .

5. The vector system for use according to any of claims 1 to 4, comprising at least two AAV particles, each of said AAV particles comprising:
a) for one, a first polynucleotide comprising an inverted terminal repeat at each end of said polynucleotide, and, between the said inverted terminal repeats, from 5' to 3': a suitable promoter followed by a partial coding sequence that contains the N-terminal part of the *Otoferlin* gene, and a splice donor site, and
b) for the other, a second polynucleotide comprising an inverted terminal repeat at each end of said polynucleotide, and, between the said inverted terminal repeats, from 5' to 3': a splice acceptor site, a partial coding sequence that contains the C-terminal part of the *Otoferlin* gene, optionally followed by a polyadenylation sequence,
wherein the said first and second polynucleotides also contain a recombinogenic sequence that is located after the splice donor site in said first polynucleotide and before the splice acceptor site in said second polynucleotide, and
wherein the coding sequences in the first and second polynucleotides when combined encode the full-length of the Otoferlin polypeptide, or a functional fragment thereof.

6. The vector system for use according to claim 5, wherein said *Otoferlin* gene has the sequence SEQ ID NO:2 or SEQ ID NO:22.

7. The vector system for use according to claim 5, wherein said N-terminal part of the *Otoferlin* gene is of SEQ ID NO:3 and said C-terminal part of the *Otoferlin* gene is of SEQ ID NO:23 or of SEQ ID NO:4.

8. The vector system for use according to any of claims 1-7, wherein said AAV particles are of the AAV2 serotype.

9. The vector system for use according to any of claims 1-8, comprising AAV2 particles in which the capsid has been modified by substituting the tyrosine amino acid residues into phenylalanine amino acid residues.

10. The vector system for use according to any of claims 1-9, wherein said human patients have been diagnosed from the DFNB9 deafness after language acquisition.

11. The vector system for use according to any of claims 1-10, wherein said patients are teenagers or adult humans suffering from DFNB9 deafness induced by thermosensitive mutations, preferably chosen from: P.Q994VfsX6, P.I515T, p.G541S, PR1607W, p.E1804del.

12. A pharmaceutical composition comprising at least two AAV particles, each of them comprising a polynucleotide comprising a partial coding sequence that encodes i) the N-terminal part of the Otoferlin polypeptide or of a functional fragment thereof, for one, and ii) the C-terminal part of the Otoferlin polypeptide or of a functional fragment thereof, for the other, , as well as a pharmaceutically acceptable vehicle, for use for treating patients suffering from DFNB9 deafness or for preventing DFNB9 deafness in patients having DFNB9 mutations, wherein said patients are human having a developed and mature auditory system, such as new born babies, toddlers, infants, teenagers or adults.

13. The pharmaceutical composition for use according to claim 12, wherein said N-terminal part of the Otoferlin gene is of SEQ ID NO:3 and said C-terminal part of the Otoferlin gene is of SEQ ID NO:4 or of SEQ ID NO:23.

14. The pharmaceutical composition for use according to claim 12 or 13, wherein said AAV particles are of the AAV2 serotype, preferably in which the capsid has been modified by substituting the tyrosine amino acid residues into phenylalanine amino acid residues.

15. The pharmaceutical composition for use according to claim 12, 13, or 14, wherein said patients are teenagers or adult humans suffering from DFNB9 deafness induced by thermosensitive mutations, preferably chosen from: P.Q994VfsX6, P.I515T, p.G541S, PR1607W, p.E1804del.

## Patentansprüche

1. Vektorsystem, umfassend mindestens zwei AAV-Partikel, von denen jedes ein Polynukleotid umfasst, umfassend eine partielle codierende Sequenz, die i) einerseits den N-terminalen Teil des Otoferlin-Polypeptids oder eines funktionellen Fragments davon und ii) andererseits den C-terminalen Teil des Otoferlin-Polypeptids oder eines funktionellen Fragments davon codiert, wobei das Vektorsystem die Expression des Otoferlin-Polypeptids voller Länge oder eines funktionellen Fragments davon in inneren Haarzellen zulässt, zur Verwendung zur Behandlung von Patienten, die an DFNB9-Taubheit leiden, oder Prävention von DFNB9-Taubheit in Patienten mit DFNB9-Mutationen, wobei die Patienten menschlich mit einem entwickelten und ausgereiften Gehör sind.

2. Vektorsystem zur Verwendung nach Anspruch 1, wobei das Otoferlin-Polypeptid die Sequenz SEQ ID Nr. 1 aufweist.

3. Vektorsystem zur Verwendung nach Anspruch 1, wobei das Otoferlin-Polypeptid die Sequenz SEQ ID Nr. 5 aufweist.

4. Vektorsystem zur Verwendung nach einem der Ansprüche 1 bis 3, wobei die menschlichen Patienten neugeborene Babys, Kleinkinder, Kinder, Jugendliche oder Erwachsene sind.

5. Vektorsystem zur Verwendung nach einem der Ansprüche 1 bis 4, umfassend mindestens zwei AAV-Partikel, wobei jedes der AAV-Partikel umfasst:
a) einerseits ein erstes Polynukleotid, umfassend eine invertierte terminale Wiederholung an jedem Ende des Polynukleotids und zwischen den invertierten terminalen Wiederholungen von 5' bis 3': einen geeigneten Promoter, auf den eine partielle codierende Sequenz folgt, die den N-terminalen Teil des *Otoferlin-*Gens enthält, und eine Spleiß-Donor-Stelle, und
b) andererseits ein zweites Polynukleotid, umfassend eine invertierte terminale Wiederholung an jedem Ende des Polynukleotids und zwischen den invertierten terminalen Wiederholungen von 5' bis 3': eine Spleiß-Akzeptor-Stelle, eine partielle codierende Sequenz, die den C-terminalen Teil des *Otoferlin-Gens* enthält, auf die optional eine Polyadenylierungssequenz folgt,
wobei das erste und das zweite Polynukleotid außerdem eine rekombinogene Sequenz enthalten, die sich hinter der Spleiß-Donor-Stelle in dem ersten Polynukleotid und vor der Spleiß-Akzeptor-Stelle in dem zweiten Polynukleotid befindet, und
wobei die codierenden Sequenzen in dem ersten und dem zweiten Polynukleotid, wenn sie kombiniert werden, die volle Länge des Otoferlin-Polypeptids oder ein funktionelles Fragment davon codieren.

6. Vektorsystem zur Verwendung nach Anspruch 5, wobei das *Otoferlin-*Gen die Sequenz SEQ ID Nr. 2 oder SEQ ID Nr. 22 aufweist.

7. Vektorsystem zur Verwendung nach Anspruch 5, wobei der N-terminale Teil des *Otoferlin-*Gens von SEQ ID Nr. 3 ist und der C-terminale Teil des *Otoferlin-Gens* von SEQ ID Nr. 23 oder von SEQ ID Nr. 4 ist.

8. Vektorsystem zur Verwendung nach einem der Ansprüche 1 bis 7, wobei die AAV-Partikel vom AAV2-Serotyp sind.

9. Vektorsystem zur Verwendung nach einem der Ansprüche 1 bis 8, umfassend AAV2-Partikel, in denen das Kapsid durch Substituieren der Tyrosin-Aminosäurereste zu Phenylalanin-Aminosäureresten modifiziert wurde.

10. Vektorsystem zur Verwendung nach einem der Ansprüche 1 bis 9, wobei die menschlichen Patienten nach Spracherwerb mit der DFNB9-Taubheit diagnostiziert wurden.

11. Vektorsystem zur Verwendung nach einem der Ansprüche 1 bis 10, wobei die Patienten Jugendliche oder erwachsene Menschen sind, die an DFNB9-Taubheit leiden, die durch wärmeempfindliche Mutationen induziert wurde, die vorzugsweise aus: P.Q994VfsX6, P.I515T, p.G541S, PR1607W, p.E1804del ausgewählt sind.

12. Pharmazeutische Zusammensetzung, umfassend mindestens zwei AAV-Partikel, von denen jedes ein Polynukleotid umfasst, umfassend eine partielle codierende Sequenz, die i) einerseits den N-terminalen Teil des Otoferlin-Polypeptids oder eines funktionellen Fragments davon und ii) andererseits den C-terminalen Teil des Otoferlin-Polypeptids oder eines funktionellen Fragments davon codiert, sowie ein pharmazeutisch unbedenkliches Vehikel, zur Verwendung zur Behandlung von Patienten, die an DFNB9-Taubheit leiden, oder zur Prävention von DFNB9-Taubheit in Patienten mit DFNB9-Mutationen, wobei die Patienten menschlich mit einem entwickelten und ausgereiften Gehör sind, wie neugeborene Babys, Kleinkinder, Kinder, Jugendliche oder Erwachsene.

13. Pharmazeutische Zusammensetzung zur Verwendung nach Anspruch 12, wobei der N-terminale Teil des Otoferlin*-*Gens von SEQ ID Nr. 3 ist und der C-terminale Teil des Otoferlin*-*Gens von SEQ ID Nr. 4 oder von SEQ ID Nr. 23 ist.

14. Pharmazeutische Zusammensetzung zur Verwendung nach Anspruch 12 oder 13, wobei die AAV-Partikel vom AAV2-Serotyp sind, vorzugsweise in denen das Kapsid durch Substituieren der Tyrosin-Aminosäurereste zu Phenylalanin-Aminosäureresten modifiziert wurde.

15. Pharmazeutische Zusammensetzung zur Verwendung nach Anspruch 12, 13 oder 14, wobei die Patienten Jugendliche oder erwachsene Menschen sind, die an DFNB9-Taubheit leiden, die durch wärmeempfindliche Mutationen induziert wurde, die vorzugsweise aus: P.Q994VfsX6, P.I515T, p.G541S, PR1607W, p.E1804del ausgewählt sind.

## Revendications

1. Système vecteur comprenant au moins deux particules d'AAV, chacune d'elles comprenant un polynucléotide comprenant une séquence codante partielle qui code i) la partie N-terminale du polypeptide d'otoferline ou d'un fragment fonctionnel de celui-ci, pour l'une, et ii) la partie C-terminale du polypeptide d'otoferline ou d'un fragment fonctionnel de celui-ci, pour l'autre, ledit système vecteur permettant l'expression du polypeptide d'otoferline de pleine longueur, ou d'un fragment fonctionnel de celui-ci, dans des cellules ciliées internes, pour une utilisation dans le traitement de patients souffrant d'une surdité liée à DFNB9 ou dans la prévention d'une surdité liée à DFNB9 chez des patients ayant des mutations de DFNB9, dans lequel lesdits patients sont des humains ayant un système auditif développé et mature.

2. Système vecteur pour une utilisation selon la revendication 1, dans lequel ledit polypeptide d'otoferline a la séquence SEQ ID NO : 1.

3. Système vecteur pour une utilisation selon la revendication 1, dans lequel ledit polypeptide d'otoferline a la séquence SEQ ID NO : 5.

4. Système vecteur pour une utilisation selon l'une quelconque des revendications 1 à 3, dans lequel lesdits patients humains sont des nouveau-nés, des nourrissons, des enfants, des adolescents ou des adultes.

5. Système vecteur pour une utilisation selon l'une quelconque des revendications 1 à 4, comprenant au moins deux particules d'AAV, chacune desdites particules de AAV comprenant :
a) pour l'une, un premier polynucléotide comprenant une répétition terminale inversée à chaque extrémité dudit polynucléotide et, entre lesdites répétitions terminales inversées, de 5' vers 3' : un promoteur adapté suivi d'une séquence codante partielle qui contient la partie N-terminale du gène d'otoferline, et un site donneur d'épissage, et
b) pour l'autre, un deuxième polynucléotide comprenant une répétition terminale inversée à chaque extrémité dudit polynucléotide et, entre lesdites répétitions terminales inversées, de 5' vers 3' : un site accepteur d'épissage, une séquence codante partielle qui contient la partie C-terminale du gène d'otoferline, éventuellement suivie d'une séquence de polyadénylation,
dans lequel lesdits premier et deuxième polynucléotides contiennent aussi une séquence recombinogène qui est située après le site donneur d'épissage dans ledit premier polynucléotide et avant le site accepteur d'épissage dans ledit deuxième polynucléotide, et
dans lequel les séquences codantes dans les premier et deuxième polynucléotides, lorsqu'elles sont combinées, codent le polypeptide d'otoferline complet, ou un fragment fonctionnel de celui-ci.

6. Système vecteur pour une utilisation selon la revendication 5, dans lequel ledit gène d'otoferline a la séquence SEQ ID NO : 2 ou SEQ ID NO : 22.

7. Système vecteur pour une utilisation selon la revendication 5, dans lequel ladite partie N-terminale du gène d'otoferline a la SEQ ID NO : 3 et ladite partie C-terminale du gène d'otoferline a la SEQ ID NO : 23 ou la SEQ ID NO : 4.

8. Système vecteur pour une utilisation selon l'une quelconque des revendications 1 à 7, dans lequel lesdites particules d'AAV sont du sérotype AAV2.

9. Système vecteur pour une utilisation selon l'une quelconque des revendications 1 à 8, comprenant des particules d'AAV2 dans lesquelles la capside a été modifiée par remplacement des résidus d'acide aminé tyrosine en résidus d'acide aminé phénylalanine.

10. Système vecteur pour une utilisation selon l'une quelconque des revendications 1 à 9, dans lequel lesdits patients humains ont été diagnostiqués avec une surdité liée à DFNB9 après l'acquisition du langage.

11. Système vecteur pour une utilisation selon l'une quelconque des revendications 1 à 10, dans lequel lesdits patients sont des adolescents ou des humains adultes souffrant d'une surdité liée à DFNB9 induite par des mutations thermosensibles, de préférence choisies parmi : P.Q994VfsX6, P.I515T, p.G541S, PR1607W, p.E1804del.

12. Composition pharmaceutique comprenant au moins deux particules d'AAV, chacune d'elles comprenant un polynucléotide comprenant une séquence codante partielle qui code i) la partie N-terminale du polypeptide d'otoferline ou d'un fragment fonctionnel de celui-ci, pour l'une, et ii) la partie C-terminale du polypeptide d'otoferline ou d'un fragment fonctionnel de celui-ci, pour l'autre, ainsi qu'un véhicule pharmaceutiquement acceptable, pour une utilisation dans le traitement de patients souffrant d'une surdité liée à DFNB9 ou dans la prévention d'une surdité liée à DFNB9 chez des patients ayant des mutations de DFNB9, dans laquelle lesdits patients sont des humains ayant un système auditif développé et mature, tels que des nouveau-nés, des nourrissons, des enfants, des adolescents ou des adultes.

13. Composition pharmaceutique pour une utilisation selon la revendication 12, dans laquelle ladite partie N-terminale du gène d'otoferline a la SEQ ID NO : 3 et ladite partie C-terminale du gène d'otoferline a la SEQ ID NO : 23 ou la SEQ ID NO : 4.

14. Composition pharmaceutique pour une utilisation la revendication 12 ou 13, dans laquelle lesdites particules d'AAV sont du sérotype AAV2, de préférence dans lesquelles la capside a été modifiée par remplacement des résidus d'acide aminé tyrosine en résidus d'acide aminé phénylalanine.

15. Composition pharmaceutique pour une utilisation la revendication 12, 13 ou 14, dans laquelle lesdits patients sont des adolescents ou des humains adultes souffrant d'une surdité liée à DFNB9 induite par des mutations thermosensibles, de préférence choisies parmi : P.Q994VfsX6, P.I515T, p.G541S, PR1607W, p.E1804del.
